(19) Europäisches Patentamt
European Patent Office
Office européen des brevets

(11) EP 1 907 415 B1

(12) **EUROPEAN PATENT SPECIFICATION**

(45) Date of publication and mention of the grant of the patent:
**28.09.2011 Bulletin 2011/39**

(21) Application number: **06780674.5**

(22) Date of filing: **23.06.2006**

(51) Int Cl.:
***C07K 14/435*** *(2006.01)*

(86) International application number:
**PCT/JP2006/313042**

(87) International publication number:
**WO 2006/137598 (28.12.2006 Gazette 2006/52)**

(54) **METHODS FOR THE ASSESSMENT OF PESTICIDAL ACTIVITY OF A SUBSTANCE COMPRISING THE MEASUREMENT OF INSECT C-JUN AMINO-TERMINAL KINASE ACTIVITY**

VERFAHREN ZUM UNTERSUCHEN AUF SCHÄDLINGSBEKÄMPFENDE AKTIVITÄT EINER SUBSTANZ, DAS DIE MESSUNG DER AKTIVITÄT EINER INSEKTEN-C-JUN-AMINO-TERMINALEN KINASE UMFASST

PROCEDE DESTINE A DOSER L'ACTIVITE ANTIPARASITAIRE D'UNE SUBSTANCE COMPRENANT LA MESURE DE L'ACTIVITE D'UNE KINASE C-JUN AMINO-TERMINALE D'INSECTE

(84) Designated Contracting States:
**AT BE BG CH CY CZ DE DK EE ES FI FR GB GR HU IE IS IT LI LT LU LV MC NL PL PT RO SE SI SK TR**

(30) Priority: **23.06.2005 JP 2005183032**

(43) Date of publication of application:
**09.04.2008 Bulletin 2008/15**

(73) Proprietor: **Sumitomo Chemical Company, Limited**
**Tokyo 104-8260 (JP)**

(72) Inventors:
- **Shimokawatoko, Yasutaka**
  **Yachiyo-shi, Chiba (JP)**
- **Craen, Marc Van De**
  **9880 (BE)**
- **Nooren, Irene**
  **3521XH (NL)**
- **Turconi, Sandra**
  **CM23 4DH (GB)**
- **Roobrouck, Annelies**
  **9700 (BE)**
- **Maddelein, Wendy**
  **9860 Gijzenzele (BE)**

(74) Representative: **Vossius & Partner**
**Siebertstrasse 4**
**81675 München (DE)**

(56) References cited:
**WO-A2-01/57022 DE-B- 1 173 720**

- **MIZUTANI T ET AL: "Involvement of the JNK-like protein of the Aedes albopictus mosquito cell line, C6/36, in phagocytosis, endocytosis and infection of West Nile virus." INSECT MOLECULAR BIOLOGY, vol. 12, no. 5, October 2003 (2003-10), pages 491-499, XP002405065 ISSN: 0962-1075**
- **WOJDA IWONA ET AL: "JNK MAP kinase is involved in the humoral immune response of the greater wax moth larvae Galleria mellonella" ARCHIVES OF INSECT BIOCHEMISTRY AND PHYSIOLOGY, vol. 56, no. 4, August 2004 (2004-08), pages 143-154, XP002403842 ISSN: 0739-4462**
- **OKUNO SHUZO ET AL: "The c-Jun N-terminal protein kinase signaling pathway mediates Bax activation and subsequent neuronal apoptosis through interaction with Bim after transient focal cerebral ischemia" JOURNAL OF NEUROSCIENCE, vol. 24, no. 36, 8 September 2004 (2004-09-08), pages 7879-7887, XP002403841 ISSN: 0270-6474**
- **DAVIES S P ET AL: "SPECIFICITY AND MECHANISM OF ACTION OF SOME COMMONLY USED PROTEIN KINASE INHIBITORS" BIOCHEMICAL JOURNAL, THE BIOCHEMICAL SOCIETY, LONDON, GB, vol. 351, no. 1, 2000, pages 95-105, XP000979108 ISSN: 0264-6021**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- BOGOYEVITCH M A ET AL: "Targeting the JNK MAPK cascade for inhibition: basic science and therapeutic potential" BIOCHIMICA ET BIOPHYSICA ACTA (BBA) - PROTEINS & PROTEOMICS, ELSEVIER, vol. 1697, no. 1-2, 11 March 2004 (2004-03-11), pages 89-101, XP004496040 ISSN: 1570-9639
- HUANG M ET AL: "INHIBITION OF NUCLEOSIDE TRANSPORT BY PROTEIN KINASE INHIBITORS" JOURNAL OF PHARMACOLOGY AND EXPERIMENTAL THERAPEUTICS, AMERICAN SOCIETY FOR PHARMACOLOGY AND, US, vol. 304, no. 2, 2003, pages 753-760, XP008036291 ISSN: 0022-3565
- WONG S ET AL: "Sole BCR-ABL inhibition is insufficient to eliminate all myeloproliferative disorder cell populations" PROCEEDINGS OF THE NATIONAL ACADEMY OF SCIENCES OF THE UNITED STATES OF AMERICA, vol. 101, no. 50, 14 December 2004 (2004-12-14), pages 17456-17461, XP002403843 ISSN: 0027-8424
- DATABASE REGISTRY [Online] 16 October 2002 (2002-10-16), XP002405642 retrieved from STN Database accession no. RN: 461661-77-8
- SLUSS H K ET AL: "A JNK SIGNAL TRANSDUCTION PATHWAY THAT MEDIATES MORPHOGENESIS AND AN IMMUNE RESPONSE IN DROSOPHILA" GENES AND DEVELOPMENT, COLD SPRING HARBOR, NY, US, vol. 10, no. 21, 1996, pages 2745-2758, XP001018620 ISSN: 0890-9369 cited in the application
- PAPADOPOULOS A I ET AL: "Effect of insecticide injection on pyruvate kinase of the insect Tenebrio molitor (Coleopteran)" PESTICIDE BIOCHEMISTRY AND PHYSIOLOGY, ACADEMIC PRESS, US, vol. 82, no. 2, June 2005 (2005-06), pages 115-124, XP004888026 ISSN: 0048-3575

SEQUENCE LISTING

<110> Sumitomo Chemical Co., Ltd.

<120> AGENT THAT MODULATES PHYSIOLOGICAL CONDITION OF PESTS, INVOLVED IN INSECT c-JUN AMINO-TERMINAL KINASE ACTIVITY

<130> S14085w001

<150> JP 2005/183032

<151> 2005-06-23

<160> 16

<210> 1
<211> 397
<212> PRT
<213> Aphis gossypii

<400> 1

Met Leu Gly Thr Gln Ser Arg Phe Val Gln Arg Pro Gln Asn Lys Pro
1 5 10 15

Asp Thr Met Thr Glu Leu Ala Asp Met Phe Tyr Thr Leu Val Phe Gly
20 25 30

Asp Thr Glu Phe Ser Val Pro Arg Arg Tyr Val Asp Leu Val Pro Lys
35 40 45

Gly Leu Gly Ala Gln Gly Met Val Val Ala Ala Met Asp Thr Ile Thr
50 55 60

Gln Gln Ser Val Ala Ile Lys Lys Leu Ser Arg Pro Phe Gln Asn Val
65 70 75 80

Thr His Ala Lys Arg Ala Tyr Arg Glu Phe Lys Leu Met Lys Leu Val
85 90 95

Asn His Lys Asn Ile Ile Gly Leu Leu Asn Ala Phe Thr Pro Gln Arg
100 105 110

Thr Leu Asp Asp Phe Gln Asp Val Tyr Leu Val Met Glu Leu Met Asp
115 120 125

Ala Asn Leu Cys Gln Val Ile Gln Met Asp Leu Asp His Glu Arg Met
130 135 140

Ser Tyr Leu Leu Tyr Gln Met Leu Cys Gly Ile Lys His Leu His Ser
145 150 155 160

Ala Gly Ile Ile His Arg Asp Leu Lys Pro Ser Asn Ile Val Val Lys
165 170 175

Ser Asp Cys Thr Leu Lys Ile Leu Asp Phe Gly Leu Ala Arg Thr Ala
180 185 190

Gly Thr Thr Phe Met Met Thr Pro Tyr Val Val Thr Arg Tyr Tyr Arg
195 200 205

Ala Pro Glu Val Ile Leu Gly Met Gly Tyr Lys Glu Asn Val Asp Ile
210 215 220

Trp Ser Val Gly Cys Ile Met Gly Glu Met Ile Arg Gly Gly Val Leu
225 230 235 240

Phe Pro Gly Thr Asp His Ile Asp Gln Trp Asn Lys Ile Ile Glu Gln
245 250 255

Leu Gly Thr Pro Ser Gln Glu Phe Met Arg Arg Leu Gln Pro Thr Val
260 265 270

Arg Asn Tyr Val Glu Asn Arg Pro Arg Tyr Pro Gly Tyr Ser Phe Asp
275 280 285

Arg Leu Phe Pro Asp Val Leu Phe Pro Ser Asp Ser Ser Glu His Asn
290 295 300

Lys Leu Lys Ala Ser Gln Ala Arg Asp Leu Leu Ser Arg Met Leu Val
305 310 315 320

Ile Asp Pro Glu Lys Arg Ile Ser Val Asp Asp Ala Leu Leu His Pro
325 330 335

Tyr Ile Asn Val Trp Tyr Asp Glu Ser Glu Val Asn Ala Pro Ala Pro

340 345 350

Gly Pro Trp Asp His Ser Val Asp Glu Arg Glu His Thr Val Asp Gln
355 360 365

Trp Lys Glu Leu Ile Tyr Gln Glu Val Met Glu Tyr Asp Pro Thr Thr
370 375 380

Ala Ala Ala Ala Ser Ala Asn Glu Pro Gln Pro Ile Arg
385 390 395

<210> 2
<211> 1194
<212> DNA
<213> Aphis gossypii

<220>
<221> CDS
<222> (1) .. (1194)

<400> 2

atg ctc gga aca caa tca agg ttt gtt caa aga cct caa aat aaa cca 48
Met Leu Gly Thr Gln Ser Arg Phe Val Gln Arg Pro Gln Asn Lys Pro
1 5 10 15

gac act atg act gaa tta gct gat atg ttc tac act tta gta ttt ggt 96
Asp Thr Met Thr Glu Leu Ala Asp Met Phe Tyr Thr Leu Val Phe Gly

20 25 30

gac act gaa ttt tca gtt ccc aga cga tat gtt gat ctt gtg ccc aaa 144
Asp Thr Glu Phe Ser Val Pro Arg Arg Tyr Val Asp Leu Val Pro Lys
35 40 45

ggt tta ggt gct cag ggc atg gtg gtt gct gct atg gac act att act 192
Gly Leu Gly Ala Gln Gly Met Val Val Ala Ala Met Asp Thr Ile Thr
50 55 60

caa caa agc gtt gca att aaa aaa cta tct aga ccc ttt caa aat gta 240
Gln Gln Ser Val Ala Ile Lys Lys Leu Ser Arg Pro Phe Gln Asn Val
65 70 75 80

aca cat gca aaa aga gca tat aga gag ttc aaa ctt atg aaa tta gta 288
Thr His Ala Lys Arg Ala Tyr Arg Glu Phe Lys Leu Met Lys Leu Val
85 90 95

aat cat aaa aat att att ggt tta cta aat gcc ttc acg cca caa agg 336
Asn His Lys Asn Ile Ile Gly Leu Leu Asn Ala Phe Thr Pro Gln Arg
100 105 110

aca tta gat gat ttt caa gat gtg tac ttg gta atg gaa ctg atg gat 384
Thr Leu Asp Asp Phe Gln Asp Val Tyr Leu Val Met Glu Leu Met Asp
115 120 125

gct aat cta tgc cag gtg atc caa atg gat ctt gat cat gaa cga atg 432
Ala Asn Leu Cys Gln Val Ile Gln Met Asp Leu Asp His Glu Arg Met

130 135 140

tca tat ctt tta tat caa atg tta tgt ggt att aaa cat tta cat tca 480
Ser Tyr Leu Leu Tyr Gln Met Leu Cys Gly Ile Lys His Leu His Ser
145 150 155 160

gca gga att att cat aga gac ctt aaa cct agt aat att gtt gta aaa 528
Ala Gly Ile Ile His Arg Asp Leu Lys Pro Ser Asn Ile Val Val Lys
165 170 175

tct gac tgc aca tta aag att ttg gat ttt gga tta gct cga aca gct 576
Ser Asp Cys Thr Leu Lys Ile Leu Asp Phe Gly Leu Ala Arg Thr Ala
180 185 190

gga acc aca ttt atg atg acg cca tat gtt gtt act aga tac tat cgt 624
Gly Thr Thr Phe Met Met Thr Pro Tyr Val Val Thr Arg Tyr Tyr Arg
195 200 205

gct cca gag gta att tta ggt atg ggt tat aag gaa aat gtt gac att 672
Ala Pro Glu Val Ile Leu Gly Met Gly Tyr Lys Glu Asn Val Asp Ile
210 215 220

tgg tca gta ggt tgt att atg ggt gag atg ata aga ggt ggc gtt tta 720
Trp Ser Val Gly Cys Ile Met Gly Glu Met Ile Arg Gly Gly Val Leu
225 230 235 240

ttt cct gga act gat cat ata gat caa tgg aat aaa att ata gaa caa 768
Phe Pro Gly Thr Asp His Ile Asp Gln Trp Asn Lys Ile Ile Glu Gln

245 250 255

ctg ggc aca cct tca caa gaa ttt atg aga aga tta caa cca act gtt 816
Leu Gly Thr Pro Ser Gln Glu Phe Met Arg Arg Leu Gln Pro Thr Val
260 265 270

aga aat tat gtt gaa aat aga cct cgt tat cca ggt tat tca ttt gat 864
Arg Asn Tyr Val Glu Asn Arg Pro Arg Tyr Pro Gly Tyr Ser Phe Asp
275 280 285

cga tta ttt cct gac gta cta ttt ccg tca gat tcg tct gaa cat aat 912
Arg Leu Phe Pro Asp Val Leu Phe Pro Ser Asp Ser Ser Glu His Asn
290 295 300

aaa ctt aaa gca agc caa gct cgt gac ctt ttg tct cga atg ctg gtt 960
Lys Leu Lys Ala Ser Gln Ala Arg Asp Leu Leu Ser Arg Met Leu Val
305 310 315 320

atc gat cca gaa aag cgt ata tct gtg gac gat gca ttg cta cat cca 1008
Ile Asp Pro Glu Lys Arg Ile Ser Val Asp Asp Ala Leu Leu His Pro
325 330 335

tat ata aat gtt tgg tat gat gaa agt gaa gta aat gca cca gca cca 1056
Tyr Ile Asn Val Trp Tyr Asp Glu Ser Glu Val Asn Ala Pro Ala Pro
340 345 350

gga cca tgg gac cat tca gta gat gaa cgt gaa cat acc gtt gat caa 1104
Gly Pro Trp Asp His Ser Val Asp Glu Arg Glu His Thr Val Asp Gln

355 360 365

tgg aaa gaa ctt atc tat caa gaa gtc atg gag tat gat cca act act 1152
Trp Lys Glu Leu Ile Tyr Gln Glu Val Met Glu Tyr Asp Pro Thr Thr
370 375 380

gca gca gca gct tct gct aat gaa cca caa cca atc cga tag 1194
Ala Ala Ala Ala Ser Ala Asn Glu Pro Gln Pro Ile Arg
385 390 395

<210> 3
<211> 29
<212> DNA
<213> Artificial Sequence

<220>
<223> Description of Artificial Sequence:Designed oligonucleotide primer for PCR

<400> 3

gtcagacatt ggacggaggt aaactgtgc 29

<210> 4
<211> 25
<212> DNA
<213> Artificial Sequence

<220>
<223> Description of Artificial Sequence:Designed oligonucleotide primer for PCR

<400> 4

gtttgttgaa tttgagattg tccac 25

<210> 5
<211> 34
<212> DNA
<213> Artificial Sequence

<220>
<223> Description of Artificial Sequence:Designed oligonucleotide primer for PCR

<400> 5

ggatccatgc tcggaacaca atcaaggttt gttc 34

<210> 6
<211> 33
<212> DNA
<213> Artificial Sequence

<220>
<223> Description of Artificial Sequence:Designed oligonucleotide primer for PCR

<400> 6

cttataaccc atacctaaaa ttacctctgg agc 33

<210> 7
<211> 33
<212> DNA
<213> Artificial Sequence

<220>
<223> Description of Artificial Sequence:Designed oligonucleotide primer for PCR

<400> 7

gctccagagg taattttagg tatgggttat aag 33

<210> 8
<211> 17
<212> DNA
<213> Artificial Sequence

<220>
<223> Description of Artificial Sequence:Designed oligonucleotide primer for PCR

<400> 8

caggaaacag ctatgac 17

<210> 9
<211> 17
<212> DNA
<213> Artificial Sequence

<220>
<223> Description of Artificial Sequence:Designed oligonucleotide primer for PCR

<400> 9

gttttcccag tcacgac 17

<210> 10
<211> 38
<212> DNA
<213> Artificial Sequence

<220>
<223> Description of Artificial Sequence:Designed oligonucleotide primer for PCR

<400> 10

cgctttgttg agtaatagtg tccatagcag caaccacc 38

<210> 11
<211> 38
<212> DNA
<213> Artificial Sequence

<220>
<223> Description of Artificial Sequence:Designed oligonucleotide primer for PCR

<400> 11

ggtggttgct gctatggaca ctattactca acaaagcg 38

<210> 12
<211> 39
<212> DNA
<213> Artificial Sequence

<220>
<223> Description of Artificial Sequence:Designed oligonucleotide primer for PCR

<400> 12.

gtggttccag ctgttcgagc taatccaaaa tccaaaatc 39

<210> 13
<211> 21
<212> PRT
<213> Artificial Sequence

<220>
<223> Description of Artificial Sequence:Designed peptide for JNK substrate

<400> 13

Lys Arg Glu Leu Val Glu Pro Leu Thr Pro Ser Gly Glu Ala Pro Asn
1 5 10 15

Gln Ala Leu Leu Arg
20

<210> 14
<211> 22
<212> PRT
<213> Artificial Sequence

<220>
<223> Description of Artificial Sequence:Designed peptide for JNK substrate

<400> 14

Lys Arg Glu Leu Val Glu Pro Leu Thr Pro Ser Gly Glu Ala Pro Asn
1 5 10 15

Gln Ala Leu Leu Arg Cys
20

<210> 15
<211> 732
<212> DNA
<213> Blattella germanica

<220>
<221> CDS
<222> (1).. (732)

<400> 15

atg ttc tac ama gtg gag ttt gga gac act gag ttc tct gtt ccc aat 48
Met Phe Tyr Xaa Val Glu Phe Gly Asp Thr Glu Phe Ser Val Pro Asn
1 5 10 15

cgc tat tcg gac ttg gca ccc aaa ggg gtg ggt gcg cag ggc atg gtc 96
Arg Tyr Ser Asp Leu Ala Pro Lys Gly Val Gly Ala Gln Gly Met Val
20 25 30

tgt gct gct tat gac aca gtg act cag cag aac gtg gct atc aag aaa 144
Cys Ala Ala Tyr Asp Thr Val Thr Gln Gln Asn Val Ala Ile Lys Lys
35 40 45

ttg agc cgt cca ttc cag aat gtt acg cat gct aag cgy gcc tat cga 192
Leu Ser Arg Pro Phe Gln Asn Val Thr His Ala Lys Xaa Ala Tyr Arg
50 55 60

gaa ttc aag ctc atg aag ctg gtt aat cac aag aat att att ggc ctg 240
Glu Phe Lys Leu Met Lys Leu Val Asn His Lys Asn Ile Ile Gly Leu
65 70 75 80

cta aat gct ttc aca cca caa aga tca tta gag gag ttt caa gat gtt 288
Leu Asn Ala Phe Thr Pro Gln Arg Ser Leu Glu Glu Phe Gln Asp Val
85 90 95

tat cta gta atg gag ctt atg gat gcc aac ctc tgc cag gtc atc caa 336
Tyr Leu Val Met Glu Leu Met Asp Ala Asn Leu Cys Gln Val Ile Gln
100 105 110

atg gac cta gat cat gag agg atg tct tat ctt ctc tat cag atg cth 384
Met Asp Leu Asp His Glu Arg Met Ser Tyr Leu Leu Tyr Gln Met Xaa
115 120 125

tgc ggc atc aag cat ctc cac tct gca ggc atc ata cat agr gac ctg 432
Cys Gly Ile Lys His Leu His Ser Ala Gly Ile Ile His Xaa Asp Leu
130 135 140

aaa mct agc aac att gtg gta aaa tca gac tgt acc ctg aaa ata tta 480
Lys Xaa Ser Asn Ile Val Val Lys Ser Asp Cys Thr Leu Lys Ile Leu
145 150 155 160

gac ttt ggt ttg gca aga acg gct ggc aca aca ttc atg atg act cca 528
Asp Phe Gly Leu Ala Arg Thr Ala Gly Thr Thr Phe Met Met Thr Pro
165 170 175

tat gtt gtt acg cgc tac tat agg gca cca gag gta ata tta ggt atg 576
Tyr Val Val Thr Arg Tyr Tyr Arg Ala Pro Glu Val Ile Leu Gly Met
180 185 190

gga tac aaa gaa aat gtg gac atc tgg tca gta ggg tgc atc atg ggt 624
Gly Tyr Lys Glu Asn Val Asp Ile Trp Ser Val Gly Cys Ile Met Gly
195 200 205

gag atg ata cga ggt gga gtg ttg ttt cca ggg aca gat cac att gat 672
Glu Met Ile Arg Gly Gly Val Leu Phe Pro Gly Thr Asp His Ile Asp
210 215 220

cag tgg aat aaa ata ata gaa caa ctt ggt act cca tca caa gac ttt 720
Gln Trp Asn Lys Ile Ile Glu Gln Leu Gly Thr Pro Ser Gln Asp Phe
225 230 235 240

agg aaa cga ctc 732
Arg Lys Arg Leu

<210> 16
<211> 244
<212> PRT
<213> Blattella germanica

<400> 16

Met Phe Tyr Xaa Val Glu Phe Gly Asp Thr Glu Phe Ser Val Pro Asn
1 5 10 15

Arg Tyr Ser Asp Leu Ala Pro Lys Gly Val Gly Ala Gln Gly Met Val
20 25 30

Cys Ala Ala Tyr Asp Thr Val Thr Gln Gln Asn Val Ala Ile Lys Lys
35 40 45

Leu Ser Arg Pro Phe Gln Asn Val Thr His Ala Lys Xaa Ala Tyr Arg
50 55 60

Glu Phe Lys Leu Met Lys Leu Val Asn His Lys Asn Ile Ile Gly Leu
65 70 75 80

Leu Asn Ala Phe Thr Pro Gln Arg Ser Leu Glu Glu Phe Gln Asp Val
85 90 95

Tyr Leu Val Met Glu Leu Met Asp Ala Asn Leu Cys Gln Val Ile Gln
100 105 110

Met Asp Leu Asp His Glu Arg Met Ser Tyr Leu Leu Tyr Gln Met Xaa
115 120 125

Cys Gly Ile Lys His Leu His Ser Ala Gly Ile Ile His Xaa Asp Leu

130 135 140

Lys Xaa Ser Asn Ile Val Val Lys Ser Asp Cys Thr Leu Lys Ile Leu
145 150 155 160

Asp Phe Gly Leu Ala Arg Thr Ala Gly Thr Thr Phe Met Met Thr Pro
165 170 175

Tyr Val Val Thr Arg Tyr Tyr Arg Ala Pro Glu Val Ile Leu Gly Met
180 185 190

Gly Tyr Lys Glu Asn Val Asp Ile Trp Ser Val Gly Cys Ile Met Gly
195 200 205

Glu Met Ile Arg Gly Gly Val Leu Phe Pro Gly Thr Asp His Ile Asp
210 215 220

Gln Trp Asn Lys Ile Ile Glu Gln Leu Gly Thr Pro Ser Gln Asp Phe
225 230 235 240

Arg Lys Arg Leu

## Description

Technical Field

The present invention relates to a method for assaying pesticidal activity of a test substance, which comprises:

(1) a first step of measuring the activity of a c-Jun $NH_2$-terminal kinase selected from the following group A in a reaction system in which said c-Jun $NH_2$-terminal kinase contacts with a test substance and the peptide of SEQ ID NO:14; and
(2) a second step of evaluating the pesticidal activity of said test substance based on the difference obtained by comparing the activity measured in the first step with the activity of a control, wherein said test substance is evaluated as having pesticidal activity when it has been identified that said test substance has an ability to inhibit the reaction of the c-Jun $NH_2$-terminal kinase with the peptide of SEQ ID NO:14:

< group A>

(a) a protein comprising the amino acid sequence of SEQ ID NO: 1;
(b) a protein comprising an amino acid sequence with deletion, addition or substitution of one or more amino acids in the amino acid sequence of SEQ ID NO: 1, wherein said protein has c-Jun $NH_2$-terminal kinase activity;
(c) a protein comprising an amino acid sequence that has sequence identity of 83% or more to the amino acid sequence of SEQ ID NO: 1, wherein said protein has c-Jun $NH_2$-terminal kinase activity;
(d) a protein comprising the amino acid sequence encoded by the nucleotide sequence of SEQ ID NO: 2;
(e) a protein comprising an amino acid sequence encoded by a nucleotide sequence that has sequence identity of 65% or more to the nucleotide sequence of SEQ ID NO: 2, wherein said protein has c-Jun $NH_2$-terminal kinase activity;
(f) a protein comprising an amino acid sequence encoded by a polynucleotide, wherein said polynucleotide hybridizes under a stringent condition to a polynucleotide comprising a nucleotide sequence complementary to the nucleotide sequence of SEQ ID NO: 2, and wherein said protein has c-Jun $NH_2$-terminal kinase activity;
(g) a protein comprising an amino acid sequence of an insect c-Jun $NH_2$-terminal kinase; and
(h) a protein comprising an amino acid sequence of a cotton aphid c-Jun $NH_2$-terminal kinase.

Background Art

Regarding insect c-Jun $NH_2$-terminal kinase, for example, it has been suggested:

that the D. melanogaster c-Jun $NH_2$-terminal kinase, encoded by the bsk gene, is involved in different physiological processes as early as the embryonic stage, and bsk mediates an immune response in cells in culture and morphogenesis in vivo (for example, Sluss et al., Genes Dev. 10 (21): 2745-58, 1996);
that the developmental signaling protein wingless (wg) and c-Jun $NH_2$-terminal kinase signaling cascades collaborate to promote both dorsal closure and ventral patterning (for example, McEven et al., Development; 127 (16): 3607-17, 2000);
that c-Jun $NH_2$-terminal kinase signaling cascades are not necessary for normal wing development, but only when a level of the signal is not normal, the cascades are necessary as information for development of wing at a normal position, this is not seen at development where c-Jun $NH_2$-terminal kinase signaling cascades are normal, but when the signaling cascades become abnormal, the kinase is activated for providing a system for maintaining suitable development (for example, Adachi-Yamadaet al., Nature, 400 (6740): 166-9, 1999), and the like.

In addition, in D. melanogaster a null mutant phenotype is described as embryonic recessive lethal, which is in accordance with central function of insect c-Jun NH2-terminal kinase. Mutations have been isolated which affect the embryonic dorsal cuticle and the embryonic dorsal epidermis.

During D. melanogaster development, the c-Jun $NH_2$-terminal kinase signal transduction pathway regulates morphogenetic tissue closure movements that involve cell shape changes and reorganization of the actin cyto-skeleton (for example, Noselli et al., Curr Opin Genet Dev., 9 (4): 466-72, 1999). Also in D. melanogaster, c-Jun NH2-terminal kinase loss-of-function mutations have been described which are lethal at the embryonic stage. (for example, Sluss et al., Genes Dev. 10 (21): 2745-58, 1996).

Discovery of agricultural chemicals has traditionally been based on a random screening process, often directly testing the effects of specific chemicals on whole organisms, such as insects, fungi and/or plants and determining

biological activity. Once chemical compounds with the appropriate biological activity are discovered, more intense research is required to specifically determine the mode of action or site of action of these compounds at the molecular level, in order to predict safety and environmental load of these compounds.

Disclosure of Invention

This invention describes a more target-based approach of screening agricultural chemicals, whereby compounds are screened against a specific target that has been identified as biologically and/or physiologically relevant with intent of chemically interfering with the target site to control insects or other pest organisms.

Specifically, it is described herein that an agent that modulates physiological condition of pests and having an ability to modulate the activity of an insect c-Jun $NH_2$-terminal kinase is useful to control pests.

Herein disclosed is:

1. An agent that modulates physiological condition of pests, wherein said agent has an ability to modulate the activity of an insect c-Jun $NH_2$-terminal kinase;
2. An agent according to item 1, wherein said c-Jun $NH_2$-terminal kinase is a cotton aphid c-Jun $NH_2$-terminal kinase;
3. An agent according to item 1, wherein said agent is a pesticidal agent;
4. An agent according to item 1, wherein said ability to modulate the activity of an insect c-Jun $NH_2$-terminal kinase is an ability to inhibit a reaction of the insect c-Jun $NH_2$-terminal kinase with the peptide of SEQ ID NO: 14;
5. A pesticidal agent which comprises a substance that has an ability to modulate the activity of an insect c-Jun $NH_2$-terminal kinase or an agriculturally acceptable salt of the substance as an active ingredient;
6. A pesticidal agent according to item 5, wherein said substance has an ability to inhibit a reaction of the insect c-Jun $NH_2$-terminal kinase with the peptide of SEQ ID NO: 14;
7. A pesticidal agent according to item 6, wherein said substance has an ability to inhibit the reaction of the insect c-Jun $NH_2$-terminal kinase with the peptide of SEQ ID NO: 14 in a cell-free system, wherein in the presence of said substance of 10 micro M or more the activity of said c-Jun $NH_2$-terminal kinase is lower than that in the absence of said substance;
8. A pesticidal agent according to item 6, wherein said substance has an ability to inhibit a reaction of the insect c-Jun $NH_2$-terminal kinase with the peptide of SEQ ID NO: 14 in a cell-free system with an IC50 of 100 micro M or less.
Furthermore, the present invention relates to the following embodiments:
9. A method for assaying pesticidal activity of a test substance, which comprises:

   (1) a first step of measuring the activity of a c-Jun $NH_2$-terminal kinase selected from the following group A in a reaction system in which said c-Jun $NH_2$-terminal kinase contacts with a test substance and the peptide of SEQ ID NO:14; and '(2) a second step of evaluating the pesticidal activity of said test substance based on the difference obtained by comparing the activity measured in the first step with the activity of a control; wherein said test substance is evaluated as having pesticidal activity when it has been identified that said test substance has an ability to inhibit the reaction of the c-Jun $NH_2$-terminal kinase with the peptide of SEQ ID NO:14:

      <group A> .

         (a) a protein comprising the amino acid sequence of SEQ ID NO: 1;
         (b) a protein comprising an amino acid sequence with deletion, addition or substitution of one or more amino acids in the amino acid sequence of SEQ ID NO: 1, wherein said protein has c-Jun $NH_2$-terminal kinase activity;
         (c) a protein comprising an amino acid sequence that has sequence identity of 83% or more to the amino acid sequence of SEQ ID NO: 1, wherein said protein has c-Jun $NH_2$-terminal kinase activity;
         (d) a protein comprising the amino acid sequence encoded by the nucleotide sequence of SEQ ID NO: 2;
         (e) a protein comprising an amino acid sequence encoded by a nucleotide sequence that has sequence identity of 65% or more to the nucleotide sequence of SEQ ID NO: 2, wherein said protein has c-Jun $NH_2$-terminal kinase activity;
         (f) a protein comprising an amino acid sequence encoded by a polynucleotide, wherein said polynucleotide hybridizes under a stringent condition to a polynucleotide comprising a nucleotide sequence complementary to the nucleotide sequence of SEQ ID NO: 2, and wherein said protein has c-Jun $NH_2$-terminal kinase activity;
         (g) a protein comprising an amino acid sequence of an insect c-Jun $NH_2$-terminal kinase; and
         (h) a protein comprising an amino acid sequence of a cotton aphid c-Jun $NH_2$-terminal kinase;

10. A method for screening a pesticidal substance, which comprises selecting a substance having the pesticidal activity that is evaluated by the method according to item 9, wherein the substance is selected (a) when the substance has an ability to inhibit the reaction of the c-Jun $NH_2$-terminal kinase with the peptide of SEQ ID NO: 14 in a cell-free system, wherein in the presence of said substance of 10 micro M or more the activity of the c-Jun $NH_2$-terminal kinase is lower than that in the absence of said substance; or (b) when the substance has an ability to inhibit the reaction of the c-Jun $NH_2$-terminal kinase with the peptide of SEQ ID NO: 14 in a cell-free system with an $IC_{50}$ of 100 micro M or less. Also disclosed herein is:
11. A pesticidal agent which comprises a substance selected by the method according to item 10 or agriculturally acceptable salts thereof as an active ingredient;
12. A method for controlling pests which comprises applying an effective amount of the pesticidal agent according to item 5, 6, 7, 8 or 11 to the pest, habitat of the pest or plant to be protected from the pest;
13. A method for controlling pests which comprises:

identifying a substance having the pesticidal activity that is evaluated by the method according to item 9, and contacting the pest with the identified pesticidal substance.
The invention relates to the following embodiments:

14. An insect c-Jun $NH_2$-terminal kinase comprising an amino acid sequence selected from the following group B:

<group B>

(a) the amino acid sequence of SEQ ID NO: 1;
(b) an amino acrid sequence with deletion, addition or substitution of one or more amino acids in the amino acid sequence - of SEQ ID NO: 1, wherein said amino acid sequence has c-Jun $NH_2$-terminal kinase activity;
(c) an amino acid sequence that has sequence identity of 95% or more to the amino acid sequence of SEQ ID NO: 1, wherein said amino acid sequence has c-Jun $NH_2$-terminal kinase activity;
(d) the amino acid sequence encoded by the nucleotide sequence of SEQ ID NO: 2;
(e) an amino acid sequence encoded by a nucleotide sequence that has sequence identity of 75 % or more to the nucleotide sequence of SEQ ID NO: 2, wherein said amino acid sequence has c-Jun $NH_2$-terminal kinase activity; and
(f) an amino acid sequence encoded by a polynucleotide, wherein said polynucleotide hybridizes under a stringent condition to a polynucleotide comprising a nucleotide sequence complementary to the nucleotide sequence of SEQ ID NO: 2, wherein said amino acid sequence has c-Jun $NH_2$-terminal kinase activity.

15. Use of an insect c-Jun $NH_2$-terminal kinase as a reagent that provides an indicator to evaluate pesticidal activity;
16. Use of an insect c-Jun $NH_2$-terminal kinase according to item 14 as a reagent that provides an indicator to evaluate pesticidal activity;
17. Use according to claim 16, wherein the c-Jun $NH_2$-terminal kinase is that of item 14;
18. A polynucleotide according to item 17, which comprises the nucleotide sequence of SEQ ID NO: 2;
19. A polynucleotide which comprises a nucleotide sequence complementary to a nucleotide sequence of a polynucleotide according to item 17 or 18;
20. A polynucleotide comprising:

a partial nucleotide sequence of a polynucleotide according to item 17 or 18 or
a nucleotide sequence complementary to said partial nucleotide sequence, which comprises a nucleotide sequence of SEQ ID NO: 3 or 4;

21. A method for obtaining a polynucleotide comprising a nucleotide sequence encoding an amino acid sequence of a c-Jun $NH_2$-terminal kinase, which comprises:

a step of amplifying a desired polynucleotide by polymerase chain reaction using as a primer a polynucleotide according to item 20;
a step of identifying the desired polynucleotide amplified; and
a step of recovering the identified polynucleotide;

22. A method for obtaining a polynucleotide comprising a nucleotide sequence encoding an amino acid sequence of a c-Jun $NH_2$-terminal kinase, which comprises:

a step of detecting a desired polynucleotide by hybridization using as a probe a polynucleotide according to item 19 or 20;
a step of identifying the desired polynucleotide detected; and
a step of recovering the identified polynucleotide;

23. A circular polynucleotide comprising a nucleotide sequence of a polynucleotide according to item 17 or 18, wherein said nucleotide sequence is operably linked to a baculovirus promoter;
24. A circular polynucleotide according to item 23, wherein said promoter is a polyhedrin gene promoter;
25. A circular polynucleotide according to item 23 or 24, wherein said polynucleotide, comprises a replication origin for autonomous replication in a host cell;
26. A circular polynucleotide according to item 23, 24 or wherein said polynucleotide comprises a nucleotide sequence of a baculovirus shuttle vector and capable of propagating as a virus in an insect cell;
27. A method for producing a circular polynucleotide, which comprises ligating a polynucleotide according to item 17 or 18 into a vector;
28. A transformant in which a polynucleotide according to item 17 or 18 is introduced, wherein the transformant is a eukaryotic or a prokaryotic cell;
29. A transformant according to item 28, wherein said transformant is a transformed insect cell;
30. A method for producing a transformant, which comprises introducing a polynucleotide according to item 17 or 18 into a host cell;
31. A recombinant baculovirus comprising within its genome a polynucleotide according to item 17 or 18;
32. A method for producing a c-Jun $NH_2$-terminal kinase, which comprises a step of culturing the transformant according to item 28 or 29 and recovering a produced c-Jun $NH_2$-terminal kinase;
33. Use of a c-Jun $NH_2$-terminal kinase according to item 15 or a polynucleotide according to any one of items 17 to 18 and 20 as an experimental tool for screening a pesticidal substance. Disclosed herein is also:
34. Use of a c-Jun $NH_2$-terminal kinase according to item 14 or a polynucleotide according to any one of items 17 to 21 as a research tool;
35. A system which comprises:

a means to input, store and manage a data information of an ability of test substances, wherein said ability is an ability to modulate the activity of an insect c-Jun $NH_2$-terminal kinase;
a means to query and retrieve the data information based on a desired criterion; and
a means to display and output the result which is queried and retrieved.

Modes for Carrying Out the Invention

The present invention will be explained in detail below.
In the present invention, the "pests" indicates small animals which cause harm or discomfort to life of the people by harming man and animals directly or by damaging crops. Examples thereof include arthropod such as insects, mites and ticks and Nematoda, and typical examples of which are as follows:

Hemiptera:

Delphacidae such as Laodelphax striatellus, Nilaparvata lugens and Sogatella furcifera, Deltocephalidae such as Nephotettix cincticeps and Empoasca onukii, Aphididae such as Aphis gossypii and Myzus persicae, Pentatomidae, Aleyrodidae such as Trialeurodes vaporariorum, Bemisia tabaci and Bemisia argentifolli, Coccidae, Tingidae, Psyllidae, etc.

Lepidoptera:

Pyralidae such as Chilo suppressalis, Cnaphalocrocis medinalis, Ostrinia nubilalis and Parapediasia teterrella, Noctuidae such as Spodoptera litura, Spodoptera exigua, Pseudaletia separata,
Mamestra brassicae, Agrotis'ipsilon, Trichoplusia spp., Heliothis spp., Helicoverpa spp. and Earias spp., Pieridae such as Pieris rapae crucivora, Tortricidae such as Adoxophyes orana fasciata,
Grapholita molesta and Cydia pomonella, Carposinidae such as Carposina niponensis, Bucculatricidae such as Lyonetia clerkella,, Gracillariidae such as Phyllonorycter ringoniella,
Phyllocnistidae such as Phyllocnistis citrella, Yponomeutidae such as Plutella xylostella, Gelechiidae such as Pectinophora gossypiella, Arctiidae, Tineidae, etc.

Diptera:

Culex such as Culex pipiens pallens, Cules tritaeniorhynchus and Culex quinguefasciatus., Aedes such as Aedes aegypti and Aedes albopictus, Anopheles such as Anophelinae sinensis, Chironomidae, Muscidae such as Musca domestica and Muscina stabulans, Calliphoridae, Sarcophagidae, Fannia canicularis, Anthomyiidae such as Delia Platura and Delia antigua, Trypetidae, Drosophilidae, Psychodidae, Simuliidae, Tabanidae, Stomoxyidae, Agromyzidae, etc.

Coleoptera:

Diabrotica such as Diabrotica virgifera virgifera and Diabrotica undecimpunctata howardi, Scarabaeidae such as Anomala cuprea and Anomala rufocuprea, Curculionidae such as Sitophilus zeamais, Lissorphoptrus oryzophilus and Calosobruchys chinensis, Tenebrionidae such as Tenebrio molitor and Tribolium castaneum, Chrysomelidae such as Oulema oryzae, Aulacophora femoralis, Phyllotreta striolata and Leptinotarsa decemlineata, Anobiidae, Epilachna spp. such as Epilachna vigintioctopunctata, Lyctidae, Bostrychidae, Cerambycidae, Paederus fuiscipes, etc.

Thysanoptera:

Thripidae such as Thrips spp. including Thrips palmi, Frankliniella spp. including Frankliniella occidentalis and Sciltothrips spp. including Sciltothrips dorsalis, Phlaeothripidae, etc.

Hymenoptera:

Tenthredinidae, Formicidae, Vespidae, etc.

Dictyoptera:

Blattidae, Blattellidae, etc.

Orthoptera:

Acrididae, Gryllotalpidae etc.

Siphonaptera:

Pulex irritans, etc.

Anoplura:

Pediculus humanus capitis, etc.

Isoptera:

Termitidae, etc.

Acarina:

Tetranychidae such as Tetranychus urticae, Tetranychus kanzawai, Panonychus citri, Panonychus ulmi, and Oligonychus spp., Eriophyidae such as Aculops pelekassi and Aculus schlechtendali, Tarsonemidae such as Polyphagotarsonemus latus, Tenuipalpidae, Tuckerellidae, Ixodidae such as Haemaphysalis longicornis, Haemaphysalisflava, Dermacentor taiwanicus, Ixodes ovatus, Ixodes persulcatus and Boophilus microplus, Acaridae such as Tyrophagus putrescentiae, Dermanyssidae, Cheyletidae such as Dermatophagoides farinae and Dermatophagoides ptrenyssnus, such as Cheyletus eruditus, Cheyletus malaccensis and Cheyletus moorei, Dermanyssus spp., etc.

Nematodes:

Pratylenchus coffeae, Pratylenchus fallax, Heterodera glycines, Globodera rostochiensis, Meloidogyne hapla, Meloidogyne incognita, etc.

The term "modulate physiological condition of pests" indicates changing condition such as various phenomena in a living body which are maintained for living in pests, for example, function such as aspiration, digestion, secretion, body liquid circulation, metabolism, neurotransmission and the like, or mechanism thereof into condition apart from usual condition. Examples include changing condition by cessation of aspiration so that oxygen necessary for internal metabolism of pests is not supplied, and changing condition by cessation of function of neurotransmission of pests so that various movements of pests are ceased.

The "agent which modulates physiological condition of pests" is an agent which can modulate physiological condition of pests when being applied to pests.

In the preset invention, the "insect c-Jun $NH_2$-terminal kinase" indicates a c-Jun NH2-terminal kinase that occurs in insect, among c-Jun $NH_2$-terminal kinase present in various organisms. Herein, insect is an animal classified under Animalia, Arthropoda, Insecta, and examples of which include arthropod of the order Protura, Collembola, Diplura, Thysanura, Ephemeroptera, Odonata, Plecoptera, Grylloblattodea, Orthoptera, Phasmatodea, Dermaptera, Mantodea, Blattaria, Isoptera, Embioptera, Psocoptera, Mallophaga, Anoplura, Thysanoptera, Hemiptera, Neuroptera, Mecoptera, Trichoptera, Lepidoptera, Coleoptera, Diptera, Hymenoptera, Siphonaptera, Strepsiptera, and the like.

C-Jun amino-terminal kinase (EC 2.7.1.37) is a protein kinase, and is a member of MAP kinase family having activity of transferring a phosphate group of ATP to c-Jun.

Several assays well known to a person skilled in the art may be used to monitor c-Jun $NH_2$-terminal kinase activity. These assays use natural substrates or synthetic peptides-substrates. For example, the c-Jun $NH_2$-terminal kinase activity can be monitored using a radioactivity based assay with biotinylated c-Jun Fusion protein as substrate. Another example to monitor the c-Jun $NH_2$-terminal kinase activity using c-Jun Fusion Protein is the Kinase-Glo Luminescent Kinase Assay. A third example to monitor the c-Jun $NH_2$-terminal kinase activity using a synthetic peptide substrate is fluorescence polarization. ATF-2 (activating transcription factor 2) is an example of a substrate of c-Jun $NH_2$-terminal kinase (Fowler Ann et al., (2002), Analytical Biochemistry 308 (2002) 223-231).

It is thought that 9-12 residues in the peptide make contact with the active site cleft in Ser/Thr protein kinases, although peptides as small as 7-mers have been shown to bind. The specificity of peptide substrates is generally restricted to residues +3 to -3 of the phosphorylation site, although residues outside of this region can occasionally contribute to substrate specificity. A peptide substrate of the kinase can be selected from sequence and and literature information of known endogenous substrates. Using the method, a plurality of different peptide substrates are designed. For example, activity on the substrate of a cotton aphid-derived c-Jun amino-terminal kinase is measured by a radioactivity-based method. From those measurement results, for example, a substrate having highest phosphate group transference activity may be selected. As such the substrate, for example, there is a peptide consisting of the amino acid sequence of SEQ ID NO: 14.

A selected peptide substrate can be used in a radioactivity-based method as described above and, for example, activity of a c-Jun $NH_2$-terminal kinase can be measured using a fluorescent polarization method using the IMAP technology. The IMAP technology is based on the high affinity binding of phosphate by immobilized metal ($M^{III}$) coordination complexes on nanoparticles. This IMAP binding reagent complexes with phosphate groups on fluorescent phosphopeptides generated in a c-Jun $NH_2$-terminal kinase reaction. Such binding causes a change in the rate of the molecular motion of the peptide, and results in an increase in the fluorescence polarization value observed for the fluorescent label attached at the end of the peptide.

Among the aforementioned various methods of measuring activity of a c-Jun $NH_2$-terminal kinase, as a method for automatic and effective measurement of the c-Jun $NH_2$-terminal kinase activity of many samples, the aforementioned fluorescent polarization method using the IMAP technology using, as a substrate, a peptide consisting of the amino acid sequence of SEQ ID NO: 14 is desirable. Examples include a method using IMAP screening Express Kit with progressive Binding system (manufactured by Molecular Device) according to a method described in the manual annexed to the system and employing, as a substrate, a peptide comprising the amino acid sequence of SEQ ID NO: 13 to which biotin was added at the amino-terminus, and a peptide comprising the amino acid sequence of SEQ ID NO: 14 in which the carboxyl-terminus was fluorescently labeled.

In addition, a method of measuring activity of an insect c-Jun $NH_2$-terminal kinase can be performed by a similar method to that described above.

Several amino acid sequences of c-Jun $NH_2$-terminal kinase have been identified in different insect species, for example in D. melanogaster (accession No. AAB51187), in Anopheles gambiae (accession No. EAA05905), in Aedes albopictus (accession No. AA031950), in Apis mellifera (accession No. XP_392806), and the like, which can be found in public databases. Also, several nucleotide sequences of c-Jun $NH_2$-terminal kinase genes have been identified in different insect species, for example in D. melanogaster (accession No. NM_164900), in Anopheles, gambiae (accession No. XM_310236), in Aedes albopictus (accession No. AF515780), in Apis mellifera (accession No.XM 392806), and the

like, which can be found in public databases.

In addition, according to the methods described below, an amino acid sequence of c-Jun $NH_2$-terminal kinase and a nucleotide sequence of c-Jun $NH_2$-terminal kinase gene can be identified from a cotton aphid. The identified amino acid sequence of cotton aphid c-Jun $NH_2$-terminal kinase is shown in SEQ ID NO: 1, and the nucleotide sequence of cotton aphid c-Jun $NH_2$-terminal kinase gene is shown in SEQ ID NO: 2.

Several amino acid sequences of c-Jun $NH_2$-terminal kinase have been identified in animals other than insect, for example in Caenorhabditis elegans (accession No. NP_741434), in Homo sapiens (accession No. NP_002744, NP_620448, NP_620446), and the like, which can be found in public databases. Also, several nucleotide sequences of c-Jun $NH_2$-terminal kinase genes have been identified in animals other than insect, for example in Caenorhabditis elegans (accession No. NM_171371), in Homo sapiens (accession No. NM_002753, NM_138982, NM_138980), and the like, which can be found in public databases.

Table 1 shows Sequence identity of the amino acid sequence of cotton aphid c-Jun $NH_2$-terminal kinase (SEQ ID NO: 1) and the nucleotide sequence of cotton aphid c-Jun $NH_2$-terminal kinase gene (SEQ ID NO: 2) with the sequence of c-Jun $NH_2$-terminal kinase and gene thereof found in other animals.

Table 1

| Origin of sequence | Identity of amino acid sequence (%) vs SEQ ID NO: 1 | Identity of nucleotide sequence (%) vs SEQ ID NO: 2 |
|---|---|---|
| *Drosophila melanogaster* | 84.0 | 71.3 |
| *Apis mellifera* | 88.4 | 69.6 |
| *Aedes albopictus* | 92.1 | 69.4 |
| *Anopheles gambiae* | 88.5 | 67.9 |
| *Ceanorhabditis elegans* | 71.8 | 60.6 |
| *Homo sapiens* JNK1 | 82.4 | 61.8 |
| *Homo sapiens* JNK2 | 80.0 | 62.9 |
| *Homo sapiens* JNK3 | 62.2 | 58.8 |

An ability to modulate the activity of an insect c-Jun $NH_2$-terminal kinase refers to an ability to increase or decrease activity of an insect c-Jun $NH_2$-terminal kinase, that is, means an ability to activate a c-Jun $NH_2$-terminal kinase, or an ability to inhibit activity of a c-Jun $NH_2$-terminal kinase. And, a test substance can be added to the reaction system for measuring c-Jun $NH_2$-terminal kinase activity to investigate influence of the test substance on the c-Jun $NH_2$-terminal kinase activity.

As a substance having an ability to inhibit the activity of a c-Jun $NH_2$-terminal kinase, SP600125 (Bennett BL et al., Proc Natl Acad Sci USA. 2001 Nov 20; 98 (24) : 13681-6.) , and the like are known.

An $IC_{50}$ value of a test substance in the reaction means a concentration of a test substance at which 50% of the activity of the reaction with no test substance is inhibited. The $IC_{50}$ value of a test substance can be determined by adding test substances of different concentrations to the c-Jun $NH_2$-terminal kinase activity measuring reaction system, measuring the c-Jun $NH_2$-terminal kinase activity (response) at each concentration of added test substance(dose), producing a dose-response curve, and calculating a concentration of the added test substance, at which the c-Jun $NH_2$-terminal kinase activity is 50% inhibited. More specifically, a dose-response curve may be produced using 4 Parameter Logistic Model or Sigmoidal Dose-Response Model:

```
f(x) = (A+((B-A)/(1+((C/x)^D)))
```

$$f(x) = A + \frac{B-A}{1+(C/x)^D}$$

to calculate the $IC_{50}$. Practically, the $IC_{50}$ value may be calculated using XLfit (manufactured by IDBS) which is a

commercially available calculating software.

An agent that has an ability to modulate the activity of an insect c-Jun $NH_2$-terminal kinase is an agent containing as an active ingredient a substance having an ability to modulate the activity of an insect c-Jun $NH_2$-terminal kinase.

The "agent that modulates physiological condition of pests, wherein the agent has an ability to modulate the activity of an insect c-Jun $NH_2$-terminal kinase" is an agent having an ability to modulate the activity of insect c-Jun $NH_2$-terminal kinase specified by the aforementioned measuring method, and means an agent that can modulate physiological condition of pests. Preferable examples of the agent include an agent in which an insect c-Jun $NH_2$-terminal kinase is a cotton aphid c-Jun $NH_2$-terminal kinase. In addition, preferable examples of the agent include an agent in which an agent that modulates physiological condition of pests is a pesticidal agent . In addition, preferable examples of the agent include an agent in which an ability to modulate the activity of an insect c-Jun $NH_2$-terminal kinase is an ability to inhibit a reaction using the peptide of SEQ ID NO: 14 as a substrate.

The "pesticidal agent" indicates an agent having an ability to control the pests.

Examples of a method for measuring an ability to control pests include, in addition to the methods disclosed in the present invention, a method of measuring pesticidal activity on the pests. Specifically, for example, the pesticidal activity can be measured according to the following method.

According to the method described in Handbook of Insect Rearing Vol.1 (Elsevier Science Publisers 1985), pp.35 to pp.36 except that a sterilized artificial feed having the following composition (Table 2) is prepared, and a solution of a test agent in DMSO is added at 0.5 % by volume of the artificial feed and is mixed, a cotton aphid is reared, the number of surviving cotton aphids is investigated after 6 days, and a controlling value is obtained according to the following equation.

Table 2

| Amino acid | (mg/100 ml) | Vitamins | (mg/100 ml) |
|---|---|---|---|
| L-Alanine | 100.0 | Ascorbic acid | 100.0 |
| L-arginine | 275.0 | Biotin | 0.1 |
| L-Asparagine | 550.0 | Calcium pantothenate | 5.0 |
| L-Aspartic acid | 140.0 | Choline chloride | 50.0 |
| L-cysteine (hydrochloride) | 40.0 | Inositol | 50.0 |
| L-glutamic acid | 140.0 | Nicotinic acid | 10.0 |
| L-glutamine | 150.0 | Thiamine | 2.5 |
| L-glycine | 80.0 | | |
| L-histidine | 80.0 | Others | (mg/100 ml) |
| L-isoleucine | 80.0 | Sucrose | 12500.0 |
| L-leucine | 80.0 | Dipotassium hydrogen phosphate | 1500.0 |
| L-lysine (hydrochloride) | 120.0 | Magnesium sulfate | 123.0 |
| L-methionine | 80.0 | Cupric chloride | 0.2 |
| L-phenylalanine | 40.0 | Ferric chloride | 11.0 |
| L-proline | 80.0 | Manganese chloride | 0.4 |
| L-serine | 80.0 | Zinc sulfate (anhydrous) | 0.8 |
| L-threonine | 140.0 | | |
| L-tryptophan | 80.0 | Adjusted to pH 6.8 | |
| L-tyrosine | 40.0 | | |
| L-valine | 80.0 | | |

$$\text{Controlling value (\%)} = \{1-(Cb \times Tai)/(Cai \times Tb)\} \times 100$$

Letters in the equation represent the following meanings.

Cb: Number of surviving worms before treatment in non-treating section
Cai: Number of surviving worms at observation in non-treated section
Tb: Number of surviving worms before treatment in non-treated section
Tai: Number of surviving worms at observation in a treated section

It may be said that a test agent exhibiting a significantly high controlling value has the pesticidal activity. More preferably, it may be determined that a test agent having the controlling value of 30% or more has substantial pesticidal activity, and it may be determined that a test agent having the controlling value of less than 30% has no substantial pesticidal activity.

The pesticidal agent contains a chemical substance having an ability to modulate the activity of insect c-Jun $NH_2$-terminal kinase or an agriculturally acceptable salt thereof as an active ingredient.

An agriculturally acceptable salt refers to a salt in such a form that preparation of a controlling agent and application of the preparation do not become impossible, and may be a salt in any form. Specifically, examples of the salt include acid addition salts with mineral acids such as hydrochloric acid, hydrobromic acid, hydriodic acid, sulfuric acid, nitric acid, and phosphoric, organic acids such as formic acid, acetic acid, propionic acid, oxalic acid, malonic acid, succinic acid, fumaric acid, maleic acid, lactic acid, malic acid, tartaric acid, citric acid, methanesulfonic acrid, and ethansulfonic acid, or acidic amino acids such as aspartic acid and glutamic acid; salts with inorganic bases such as sodium, potassium, magnesium, and aluminum, organic bases such as methylamine, ethylamine, and ethanolamine, or basic amino acids with lysine and ornithine; and an ammonium salts.

The "pesticidal agent which comprises a substance having an ability to modulate the activity of an insect c-Jun $NH_2$-terminal kinase or a an agriculturally acceptable salt thereof as an active ingredient" means an agent which can control pests by containing a substance having an ability to modulate the activity of insect c-Jun $NH_2$-terminal kinase identified in the measuring method or an agriculturally acceptable salt thereof as an active ingredient. Preferable examples of the substance include a compound having an ability to inhibit a reaction of a c-Jun $NH_2$-terminal kinase with the peptide of SEQ ID NO: 14. More preferable examples of the substance include a substance having an ability to inhibit the reaction of the insect c-Jun $NH_2$-terminal kinase with the peptide of SEQ ID NO: 14 in a cell-free system, wherein in the presence of the substance of 10 $\mu$M or more the activity of the c-Jun $NH_2$-terminal kinase is lower than that in the absence of the substance. In addition, further preferable examples of the substance include a substance having an ability to inhibit a reaction of the insect c-Jun $NH_2$-terminal kinase with the peptide of SEQ ID NO: 14 in a cell-free system with an IC50 of 100 $\mu$M or less.

In the present invention, the "method for assaying pesticidal activity of a test substance, which comprises a first step of measuring the activity of a c-Jun $NH_2$-terminal kinase selected from the group A in a reaction system in which the c-Jun $NH_2$-terminal kinase contacts with a test substance, and a second step of evaluating the pestcidal activity of the test substance based on the difference obtained by comparing the activity measured in the first step with the activity of a control" indicates a method characterized by comprising the first step and the second step in various methods for assaying a pesticidal ability of a test substance. The test substance is evaluated as having pesticidal activity when it has been identified that said test substance has an ability to inhibit the reaction of the c-Jun $NH_2$-terminal kinase with the peptide of SEQ ID NO:14:

Herein, the group A indicates:

(a) a protein comprising the amino acid sequence of SEQ ID NO: 1;
(b) a protein comprising an amino acid sequence with deletion, addition or substitution of one or more amino acids in the amino acid sequence of SEQ ID NO: 1, wherein said protein has c-Jun $NH_2$-terminal kinase activity;
(c) a protein comprising an amino acid sequence that has sequence identity of 83% or more to the amino acid sequence of SEQ ID NO: 1, wherein said protein has c-Jun $NH_2$-terminal kinase activity;
(d) a protein comprising the amino acid sequence encoded by the nucleotide sequence of SEQ ID NO: 2;
(e) a protein comprising an amino acid sequence encoded by a nucleotide sequence that has sequence identity of 65% or more to the nucleotide sequence of SEQ ID NO: 2, wherein said protein has c-Jun $NH_2$-terminal kinase activity;
(f) a protein comprising an amino acid sequence encoded by a polynucleotide, wherein said polynucleotide hybridizes under a stringent condition to a polynucleotide comprising a nucleotide sequence complementary

to the nucleotide sequence of SEQ ID NO: 2, and wherein said protein has c-Jun $NH_2$-terminal kinase activity;
(g) a protein comprising an amino acid sequence of an insect c-Jun $NH_2$-terminal kinase; and
(h) a protein comprising an amino acid sequence of a cotton aphid c-Jun $NH_2$-terminal kinase.

The first step is a step of measuring the activity of a c-Jun $NH_2$-terminal kinase in the state where a c-Jun $NH_2$-terminal kinase is contacted with a test substance by adding the test substance to the aforementioned various c-Jun $NH_2$-terminal kinase activity measuring reaction systems. In addition, the second step is a step of comparing the activity at measurement of a test substance with the substance of a control, and evaluating a pesticidal ability based on the difference. Herein, a control means, for example, in the case where a test substance dissolved in a solvent is added to the reaction system, a test section in which only a solvent same as that used to dissolve the test substance is added.

A c-Jun $NH_2$-terminal kinase used in a method for assaying a pestcidal ability possessed by a test substance, having the first step and the second step, is a protein shown in the group A. Among proteins of the group A, a difference which can be recognized between an amino acid sequence of protein represented by (a) and amino acid sequences of proteins represented by (b), (c), (e), (f), (g) and (h) is deletion, substitution, addition or the like of a part of amino acids. These include, for example, deletion due to processing which the protein having an amino acid sequence represented by (a) undergoes in a cell. In addition, examples include deletion, substitution, addition and the like of an amino acid generated by naturally occurring gene mutation due to a spices difference or an individual difference of an organism from which the protein is derived, or gene mutation which is artificially introduced by a site-directed mutagenesis, a random mutagenesis, mutation treatment or the like.

The number of amino acids undergoing the deletion, substitution, addition or the like may be the number in a range that the peptidase activity of a c-Jun $NH_2$-terminal kinase can be found out. In addition, examples of substitution of an amino acid include substitution with an amino acid which is similar in characteristic in hydrophobicity, charge, pH and steric structure. Specific examples of the substitution include substitution in an group of (1) glycine, alanine; (2) valine, isoleucine, leucine; (3) aspartic acid, glutamic acid, asparagine, glutamine, (4) serine, threonine; (5) lysine, arginine; (6) phenylalanine, tyrosine and the like.

Examples of a procedure of artificially introducing the deletion, addition or substitution of an amino acid (hereinafter, collectively referred to as alteration of amino acid in some cases) include a procedure of introducing site-directed mutation into a DNA encoding an amino acid sequence represented by (a) and, thereafter, expressing this DNA by a conventional method. Herein, examples of a site-directed mutagenesis include a method utilizing amber mutation (gapped duplex method, Nucleic Acids Res., 12, 9441-9456(1984)), a method by PCR using primers for mutation introduction, and the like. In addition, examples of a procedure of artificially altering an amino acid include a procedure of randomly introducing mutation into a DNA encoding an amino acid sequence represented by (a) and, thereafter, expressing this DNA by a conventional method. Herein, examples of a method of randomly introducing mutation include a method of performing PCR using a DNA encoding any of the aforementioned amino acid sequences as a template, and using a primer pair which can amplify each full length DNA at reaction condition under which an addition amount of each of dATP, dTTP, dGTP and dCTP used as a substrate is changed from a conventional concentration, or at reaction condition under which a concentration of $Mg^{2+}$ promoting a polymerase reaction is increased from a conventional concentration. Examples of the procedure of PCR include a method described, for example, in Method in Molecular Biology, (31), 1994, 97-112. Another example includes a method described in WO 0009682.

Herein, the "sequence identity" refers to identity between two nucleotide sequences or two amino acids. The "sequence identity" is determined by comparing two sequences, which are aligned in the optimal state over an all region of sequences to be compared. Herein, in optimal alignment of nucleotide sequences or amino acid sequences to be compared, addition or deletion (e.g. gap and the like) may be permitted. The sequence identity can be calculated by performing homology analysis to produce alignment using a program such as FASTA[Pearson & Lipman, Proc. Natl. Acad. Sci. USA,4, 2444-2448(1988) ], BLAST[Altschul et al., Journal of Molecular Biology, 215, 403-410(1990)], CLUSTAL W[Thompson, Higgins&Gibson, Nucleic Acid Research, 22, 4673-4680(1994a)] and the like. The program is generally available at the website (http://www.ddbj.nig.ac.jp) of DNA Data Bank of Japan [International DNA Data Bank managed in National Institute of Genetics, Center for Information Biology and DNA Data Bank of Japan ;CIB/DDBJ]. Alternatively, sequence identity can be also obtained using a commercially available sequence analyzing software. Specifically, for example, sequence identity can be calculated by performing homology analysis using GENETYX-WIN Ver. 5 (manufactured by Software Development Co.Ltd.) by a Lipman-Pearson method [Lipman, D. J. and Pearson, W.R., Science, 227, 1435-1441,(1985)] and producing alignment.

Examples of the "stringent condition" described in (f) include condition under which, in hybridization performed according to a conventional method described in Sambrook J., Frisch E. F., Maniatis T., Molecular Cloning 2nd edition, Cold Spring Harbor Laboratory press, for example, a hybrid is formed at 45˚C in a solution containing 6xSSC (a solution containing 1.5 m NaCl and 0.15 m trisodium citrate is 10xSSC) and, thereafter, this is washed with 2xSSC at 50˚C (Molecular Biology, John Wiley & Sons, N. Y. (1989), 6.3.1-6.3.6). A salt concentration in a washing step can be selected from condition from 2xSSC (low stringent condition) to 0.2xSSC (high stringent condition). A temperature in a washing

step can be selected, for example, from condition from room temperature (low stringent condition) to 65˚C (high stringent condition). Alternatively, both of a salt concentration and a temperature can be changed.

A protein described in (h) indicates a c-Jun $NH_2$-terminal kinase presents in a cotton aphid among an insect c-Jun $NH_2$-terminal kinase, and includes a protein comprising an amino acid sequence described in (a).

In addition, a protein of the group A includes a protein comprising an amino acid sequence of an insect c-Jun $NH_2$-terminal kinase described in (g) and, more preferably includes, a protein in which when aligned with the amino acid sequence of SEQ ID NO: 1 so that maximum sequence identity is obtained, amino acid residues at positions corresponding to (I) position 64, (II) position 66, (III) position 90, (IV) position 107, (V) position 112, (VI) position 195, (VII) position 288, (VIII) position 289, (IX) position 358, (X) position 359 and (XI) position 368 of the amino acid sequence of SEQ ID NO: 1 are (I) threonine (at position corresponding to 64), (II) glutamine (at position corresponding to 66), (III) phenylalanine (at position corresponding to 90), (IV) Alanine (at position corresponding to 107), (V) arginine (at position corresponding to 112), (VI) threonine (at position corresponding to 195), (VII) Aspartic acid (at position corresponding to 288), (VIII) arginine (at position corresponding to 289), (IX) serine (at position corresponding to 358), (X)valine (at position corresponding to 359) and (XI) glutamine (at position corresponding to 368), respectively. Herein, the "aligned with the amino acid sequence of SEQ ID NO: 1 so that maximum sequence identity is obtained" means that sequence identity of a plurality of amino acid sequences to be analyzed including the amino acid sequence of SEQ ID NO: 1 is analyzed by a program such as FASTA, BLAST, CLUSTAL W described above, and they are aligned. By aligning a plurality of sequences by the method, positions of conserved amino acid residues in each amino acid sequence can be determined regardless of insertion or deletion in an amino acid sequence. It is thought that conserved amino acid residues are present at the same position in the three-dimensional structure of the proteins of interest, and it is presumed that similar effect is possessed regarding specific function of the proteins of interest. For example, when insect c-Jun $NH_2$-termjnal kinase including the c-Jun $NH_2$-terminal kinase sequence of which is disclosed in the present invention, are aligned with the amino acid sequence of SEQ ID NO: 1 so that maximum sequence identity of amino acid sequences is obtained, it is shown that amino acid residues at positions corresponding to (I) position 64, (II) position 66, (III) position 90, (IV) position 107, (V) position 112, (VI) position 195, (VII) position 288, (VIII) position 289, (IX) position 358, (X) position 359 and (XI) position 368 of the amino acid sequence of SEQ ID NO: 1 are (I) threonine (at position corresponding to 64), (II) glutamine (at position corresponding to 66), (III) phenylalanine (at position corresponding to 90), (IV) Alanine (at position corresponding to 107), (V) arginine (at position corresponding to 112), (VI) threonine (at position corresponding to 195), (VII) Aspartic acid (at position corresponding to 288), (VIII) arginine (at position corresponding to 289), (IX) serine (at position corresponding to 358), (X)valine (at position corresponding to 359) and (XI) glutamine (at position corresponding to 368), respectively.

Also provided herein is a method for screening a pesticidal substance, which comprises selecting a substance having the pesticidal activity that is evaluated by the herein above described method for assaying pesticidal activity, wherein the substance is selected (a) when the substance has an ability to inhibit the reaction of the c-Jun $NH_2$-terminal kinase with the peptide of SEQ ID NO: 14 in a cell-free system, wherein in the presence of said substance of 10 micro M or more the activity of the c-Jun $NH_2$-terminal kinase is lower than that in the absence of said substance; or (b) when the substance has an ability to inhibit the reaction of the c-Jun $NH_2$-terminal kinase with the peptide of SEQ ID NO: 14 in a cell-free system with an $IC_{50}$ of 100 micro M or less.

A substance having a pesticidal ability can be screened by using a method of assaying a pesticidal ability by measuring a pesticidal ability or controlling effect on the aforementioned pests.

Alternatively, a substance having a pesticidal ability can be also screened by the method of assaying a pesticidal ability using a c-Jun $NH_2$-terminal kinase. Specifically, when it has been identified that a pesticidal ability of a test substance is a certain value or more, or a certain value or less using the method of assaying a pesticidal ability using a c-Jun $NH_2$-terminal kinase, a substance having a pesticidal ability can be screened by selecting the substance.

Since a substance selected by the screening method has a pesticidal ability, it can be used as a pesticidal agent containing the substance or an agriculturally acceptable salt as an active ingredient.

Control of pests can be usually performed by application an effective amount of a pesticidal agent to a crop protected, a pest, or a habitat of a pest.

When a pesticidal agent is used for agriculture and forestry, its application amount is usually 0.1 to 1000g in terms of an amount of a pesticidal agent per 1000 $m^2$. When a pesticidal agent is formulated into an emulsion, a water-dispersible powder, a flowable preparation, a microcapsule preparation or the like, the agent is usually applied by diluting with water to an active ingredient concentration of 1 to 10,000 ppm, and spraying this and, when a pesticidal agent is formulated into a granule, a powder or the like, the agent is usually applied as it is.

A pesticidal agent can be used by foliage-treating a plant such as a crop and the like which should be protected from pests, and can be also used by treating a seedbed before a plantlet of a crop is transplanted, or a planting hole or a strain base at planting. Further, for the purpose of controlling pests habiting a soil of a cultivating land, the agent may be used by treating the soil. Alternatively, the agent may be used by a method of winding a resin preparation which has been processed to a sheet or a string, on a crop, stretching the preparation near a crop and/or spreading on a soil

surface of a strain base.

When a pesticidal agent is used as a pest controlling agent for preventing an epidemic, an emulsion, a water-dispersible powder, a flowable or the like is usually applied by diluting with water so that an active ingredient concentration becomes 0.01 to 10,000 ppm, and an oily agent, an aerosol, a fumigant, a poison bait or the like is applied as it is.

Examples of one utility of a pesticidal agent include control of an external parasite of a livestock such as cattle, sheep, goat, and chicken, or a small animal such as dog, cat, rat, and mouse, in this case, the agent can be administered to an animal by the veterinarily known method. As a specific administration method, when systemic control is intended, the agent is administered, for example, by a tablet, mixing in feed, suppository, injection (intramuscular, subcutaneous, intravenous, intraperitoneal etc.) and the like, when non-systemic control is intended, the agent is used by a method of spraying an oily agent or an aqueous liquid agent, performing pour on or spot on treatment, washing an animal with a shampoo preparation or attaching a resin preparation which has been processed into a necklace or a ear tag to an animal. An amount of a pesticidal agent when administered to an animal body is usually in a range of 0.1 to 1,000 mg as expressed by total amount of a compound A and a compound B per 1kg of an animal.

An application amount and an application concentration of them are both different depending on the situations such as a kind of a preparation, an application time, an application place, an application method, a kind of a pest, a damage degree and the like, can be increased or decreased regardless of the aforementioned range, and can be appropriately selected.

The aforementioned pesticidal agent can be used in the method of controlling pests as described above.

In addition, on the other hand, a pest can be also controlled by identifying a substance having a pesticidal ability evaluated by the aforementioned method of assaying a pesticidal ability possessed by a pest substance, having a first step and a second step using a c-Jun $NH_2$-terminal kinase selected from group A, and contacting the identified substance having a pesticidal ability with a pest. Herein, as a method of contacting an identified substance having a pesticidal ability with a pest, the aforementioned preparation method, application method and the like can be used.

An amino acid sequence shown in the group B is an amino acid sequence of insect c-Jun $NH_2$-terminal kinase comprising any amino acid sequence of the following (a) to (g).

(a) the amino acid sequence of SEQ ID NO: 1;
(b) an amino acid sequence with deletion, addition or substitution of one or more amino acids in the amino acid sequence of SEQ ID NO: 1, wherein said amino acid sequence has c-Jun $NH_2$-terminal kinase activity;
(c) an amino acid sequence that has sequence identity of 95% or more to the amino acid sequence of SEQ ID NO: 1, wherein said amino acid sequence has c-Jun $NH_2$-terminal kinase activity;
(d) the amino acid sequence encoded by the nucleotide sequence of SEQ ID NO: 2;
(e) an amino acid sequence encoded by a nucleotide sequence that has sequence identity of 75 % or more to the nucleotide sequence of SEQ ID NO: 2, wherein said amino acid sequence has c-Jun $NH_2$-terminal kinase activity; and
(f) an amino acid sequence encoded by a polynucleotide, wherein said polynucleotide hybridizes under a stringent condition to a polynucleotide comprising a nucleotide sequence complementary to the nucleotide sequence of SEQ ID NO: 2, wherein said amino acid sequence has c-Jun $NH_2$-terminal kinase activity.

Among amino acid sequences of the group B, a difference which can be recognized between an amino acid sequence represented by (a) and amino acid sequences represented by (b), (c), (e), (f) and (g) is deletion, substitution, addition or the like of a part of amino acids. These include, for example, deletion due to processing which the protein having an amino acid sequence represented by (a) undergoes in a cell. In addition, examples include deletion, substitution, addition and the like of an amino acid generated by naturally occurring gene mutation due to a spices difference or an individual difference of an organism from which the protein is derived, or gene mutation which is artificially introduced by a site-directed mutagenesis, a random mutagenesis, mutation treatment or the like.

The number of amino acids undergoing the deletion, substitution, addition or the like may be the number in a range that the peptidase activity of a c-Jun $NH_2$-terminal kinase can be found out. In addition, examples of substitution of an amino acid include substitution with an amino acid which is similar in characteristic in hydrophobicity, charge, pH and steric structure. Specific examples of the substitution include substitution in an group of (1) glycine, alanine; (2) valine, isoleucine, leucine; (3) aspartic acid, glutamic acid, asparagine, glutamine, (4) serine, threonine; (5) lysine, arginine; (6) phenylalanine, tyrosine and the like.

Examples of a procedure of artificially introducing the deletion, addition or substitution of an amino acid (hereinafter, collectively referred to as alteration of amino acid in some cases) include a procedure of introducing site-directed mutation into a DNA encoding an amino acid sequence represented by (a) and, thereafter, expressing this DNA by a conventional method. Herein, examples of a site-directed mutagenesis include a method utilizing amber mutation (gapped duplex method, Nucleic Acids Res., 12, 9441-9456(1984)), a method by PCR using primers for mutation introduction, and the like. In addition, examples of a procedure of artificially altering an amino acid include a procedure of randomly introducing mutation into a DNA encoding an amino acid sequence represented by (a) and, thereafter, expressing this

DNA by a conventional method. Herein, examples of a method of randomly introducing mutation include a method of performing PCR using a DNA encoding any of the aforementioned amino acid sequences as a template, and using a primer pair which can amplify each full length DNA at reaction condition under which an addition amount of each of dATP, dTTP, dGTP and dCTP used as a substrate is changed from a conventional concentration, or at reaction condition under which a concentration of $Mg^{2+}$ promoting a polymerase reaction is increased from a conventional concentration. Examples of the procedure of PCR include a method described, for example, in Method in Molecular Biology, (31), 1994, 97-112. Another example includes a method described in WO 0009682.

Herein, the "sequence identity" refers to identity between two nucleotide sequences or two amino acids. The "sequence identity" is determined by comparing two sequences which are aligned in the optimal state over an all region of sequences to be compared. Herein, in optimal alignment of nucleotide sequences or amino acid sequences to be compared, addition or deletion (e.g. gap and the like) may be permitted. The sequence identity can be calculated by performing homology analysis to produce alignment using a program such as FASTA[Pearson & Lipman, Proc. Natl. Acad. Sci. USA,4, 2444-2448(1988) ], BLAST[Altschul et al., Journal of Molecular Biology, 215, 403-410(1990) ], CLUSTAL W[Thompson, Higgins&Gibson, Nucleic Acid Research, 22, 4673-4680(1994a)] and the like. The program is generally available at the website (http://www.ddbj.nig.ac.jp) of DNA Data Bank of Japan [International DNA Data Bank managed in National Institute of Genetics, Center for Information Biology and DNA Data Bank of Japan ;CIB/DDBJ]. Alternatively, sequence identity can be also obtained using a commercially available sequence analyzing software. Specifically, for example, sequence identity can be calculated by performing homology analysis using GENETYX-WIN Ver. 5 (manufactured by Software Development Co.Ltd.) by a Lipman-Pearson method [Lipman, D. J. and Pearson, W.R., Science, 227, 1435-1441,(1985)] and producing alignment.

Examples of the "stringent condition" described in (f) include condition under which, in hybridization performed according to a conventional method described in Sambrook J. , Frisch E. F., Maniatis T., Molecular Cloning 2nd edition, Cold Spring Harbor Laboratory press, for example, a hybrid is formed at 45˚C in a solution containing 6xSSC (a solution containing 1.5 m NaCl and 0.15 m trisodium citrate is 10×SSC) and, thereafter, this is washed with 2xSSC at 50˚C (Molecular Biology, John Wiley & Sons, N. Y. (1989), 6.3.1-6.3.6). A salt concentration in a washing step can be selected from condition from 2xSSC (low stringent condition) to 0.2×SSC (high stringent condition). A temperature in a washing step can be selected, for example, from condition from room temperature (low stringent condition) to 65˚C (high stringent condition). Alternatively, both of a salt concentration and a temperature can be changed.

A protein having an amino acid sequence described in (g) indicates a c-Jun $NH_2$-terminal kinase presents in a cotton aphid among an insect c-Jun $NH_2$-terminal kinase, and includes a protein comprising an amino acid sequence described in (a).

A protein having an amino acid sequence shown in the group B can be prepared, for example, according to a method described later using a polynucleotide encoding an amino acid sequence shown in the group B.

An insect c-Jun $NH_2$-terminal kinase can be used as a reagent that provides an indicator to evaluate a pesticidal activity. Specifically, for example, an insect c-Jun $NH_2$-terminal kinase can be used as a reagent that provides an indicator to evaluate a pesticidal activity by using as a c-Jun $NH_2$-terminal kinase used in the method of assaying a pesticidal ability using a c-Jun $NH_2$-terminal kinase. In addition, a more specific method can be performed according to the aforementioned method of measuring, the activity of a c-Jun $NH_2$-terminal kinase.

In addition, when an insect c-Jun $NH_2$-terminal kinase is used as a reagent that provides an indicator to evaluate a pesticidal activity, more preferably, it is desirable that an insect c-Jun $NH_2$-terminal kinase is a c-Jun $NH_2$-terminal kinase having an amino acid sequence shown in the group B.

A polynucleotide having a nucleotide sequence encoding an amino acid sequence shown in the group B (hereinafter, referred to as polynucleotide group B in some cases) has a nucleotide sequence from which a protein having an amino acid sequence can be produced shown in the group B, in a cell of an organism or an in vitro translation system. A polynucleotide group B may be a DNA cloned from a nature, a DNA in which deletion, substitution or addition of a nucleotide is introduced into a DNA cloned from a nature, for example, by a site-directed mutagenesis or a random mutagenesis, or an artificially synthesized DNA. Specifically, examples include a polynucleotide comprising a nucleotide sequence represented by SEQ ID NO: 2.

<First obtaining method>

For example, a method of obtaining a polynucleotide comprising the nucleotide sequence of SEQ ID NO: 2 included in the polynucleotide group B will be shown below. As a step, total RNA is obtained from cotton aphids, cDNA library is synthesized, and PCR amplification is performed, thereby, a polynucleotide of interest can be obtained.

A population of adults and larvae of *Aphis gossypii,* which have been reared on leaves of potted cucumber, is scraped from the surface of the leaves with a small brush, and 630 mg of the obtained population is crushed into a powder in liquid nitrogen using a mortar and a pestle. From the resulting frozen crushed powder, RNA is isolated using a RNA extracting reagent ISOGEN (manufactured by Nippon Gene) as follows. After 10 ml of ISOGEN is added to the

frozen crushed powder in the mortar, the crushed powder is ground for 10 minutes while kept on ice. After grinding, a fluid sample is transferred to a 15 ml tube with a pipette, and 2ml of chloroform (manufactured by Wako Pure Chemical Industries, Ltd.) is added thereto. Immediately, the mixture is vigorously shaken for 15 seconds and then left at rest at room temperature for 3 minutes. Then, the resulting mixture is centrifuged at 12,000×g at 4˚C for 15 minutes, and each 5 ml of aqueous layer are transferred to two new tubes. After 5 ml of ISOGEN is added to each tube, the mixture was immediately shaken vigorously for 15 seconds, and left at rest at room temperature for 3 minutes. Then, the resulting mixture is centrifuged at 12,000×g at 4˚C for 15 minutes, and each 10 ml of aqueous layer are transferred to new 50 ml tubes, respectively. Subsequently, 10 ml of isopropanol (manufactured by Wako Pure Chemical Industries, Ltd.) is added to each tube, and the mixture is kept on ice for 30 minutes. The resulting mixture is centrifuged at 12,000×g at 4˚C for 10 minutes to precipitate RNA. After the supernatant is removed, 20 ml of 70% ethanol is added to the residue. The resulting mixture is centrifuged at 10,000×g at 4˚C for 5 minutes. After the supernatant is removed, the precipitate of total RNA is slightly dried and then dissolved in 1 ml of commercially available RNase-free water (Nacalai Tesque, Inc.). An absorbance of the prepared total RNA is measured at 260 nm to calculate a concentration according to a conventional method.

RT-PCR is performed employing total RNA of cotton aphid obtained by the aforementioned method as a template, and using random primers (manufactured by Invitrogen) and superscript III (manufactured by Invitrogen) according to the manual annexed to the reagent, to synthesized a cDNA library.

PCR is performed employing cDNA library of cotton aphid obtained by the aforementioned method as a template, and using an oligonucleotide primer comprising the nucleotide sequence of SEQ ID NO: 3 and an oligonucleotide primer comprising the nucleotide sequence of SEQ ID NO: 4 as well as a Pfu Turbo (manufactured by Stratagene) according to the manual annexed to the reagent. The conditions of the PCR are as-follows: incubation at 94˚C for 10 minutes; followed by 30 cycles of PCR, one cycle being 94˚C for 30 seconds, 50˚C for 30 seconds and 72˚C for 2 minutes; and followed by incubation at 72˚C for 7 minutes.

As described above, a polynucleotide comprising the nucleotide sequence of SEQ ID NO: 2 can be obtained.

<Second obtaining method>

Alternatively, a polynucleotide shown in the polynucleotide group B can be also obtained by preparing a polynucleotide with mutation introduced therein by a method utilizing amber mutation which is the aforementioned site-directed mutagenesis, a method by PCR using a primer for introducing mutation or the like, using as a template a polynucleotide comprising the nucleotide sequence of SEQ ID NO: 2.

<Third obtaining method>

Alternatively, a polynucleotide shown in the polynucleotide group B can be also obtained by a hybridization method using a polynucleotide comprising the nucleotide sequence of SEQ ID NO: 2 as a probe. More specifically, the third obtaining method can be performed according to a conventional hybridization described in the aforementioned Sambrook J., Frisch E. F., Maniatis T., Molecular Cloning 2nd edition, published by Cold Spring Harbor Laboratory press.

<Fourth obtaining method>

Alternatively, a polynucleotide shown in the polynucleotide group B can be also obtained by preparing a primer based on an amino acid sequence of the known insect c-Jun $NH_2$-terminal kinase and performing PCR. For isolation of homologues of c-Jun $NH_2$-terminal kinase gene from other insect species such as German cockroach *(Blatella germanica),* degenerate primers are designed using Codehop program (publicly accessible on the website of Blocks Protein Analysis Server operated within the Fred Hutchinson Cancer Research Center at http://blocks.fhcrc.org/blocks/codehop.html), and based on the sequence of the aforementioned cotton aphid-derived c-Jun $NH_2$-terminal kinase gene and the previously-known nucleotide sequences of Ancylostoma caninum (NCBI accession number AAF00539), Xenopus laevis (BAB91438, BAB85483), Homo sapiens (NP_620448, NUP_002744), Mus musculus (BAC31240), Rattus norvegicus (S43969), Gallus gallus (BAA19188), Drosophila melanogaster (AAF52883, AAC47325), Anopheles gambiae (EAA05905) and Aedes albopictus(AA031950).

Partial sequences of a homologue of c-Jun $NH_2$-terminal kinase gene in a selected insect species are amplified by a series of PCR using first-strand cDNA derived from the insect species as a template. Herein, the first-strand cDNA as a template is prepared by the aforementioned method using Superscript III. Amplification by PCR is performed using a set of degenerate primers as a forward primer and a reverse primer as well as Amplitaq Gold (manufactured by Applied Biosystems) according to the manufacturer's procedure annexed to the reagent. PCR conditions are 94˚C for 10 minutes; followed by 40 cycles of PCR, one cycle being 94˚C for 30 seconds, 45˚C for 1 minute, and 72˚C for 1 minute per 1kb of a length of a predicted amplification product; and followed by 72˚C for 7 minutes. The PCR product is analyzed and

purified by agarose gel electrophoresis to obtain DNA of interest. Further, the obtained DNA is cloned into the pCR-XL-TOPO vector (manufactured by Invitrogen), and sequenced.

Then, primers specific for the resulting partial sequences of the insect homologue of c-Jun $NH_2$-terminal kinase gene are designed, and 3'RACE PCR or 5'RACE PCR is performed in order to obtain a full-length sequence of the gene. 3'and 5'RACE PCRs are performed employing first-strand cDNA prepared from the insect total RNA as a template and using SMART PCR cDNA Synthesis Kit (manufactured by Clontech) according to the manufacturer's instructions annexed to the kit.

In 3'RACE and 5'RACE reactions, universal primer mix (UPM) contained in SMART PCR cDNA Synthesis Kit is used in combination with a forward primer or a reverse primer which is specific for the sequence of interest. PCR conditions are 1 cycle at 94˚C for 10 minutes; followed by 40 cycles of PCR, one cycle being 94˚C for 15 seconds, 63˚C for 15 seconds, and 72˚C for 1 minute per 1kb of a length of a predicted amplification product; and followed by 1 cycle at 72˚C for 7 minutes. The resulting PCR product is analyzed and purified by agarose gel electrophoresis to obtain DNA of interest. Further, the obtained DNA is cloned into the pCR-XL-TOPO vector (manufactured by Invitrogen), and sequenced.

When a distinct amplification product is not obtained by the first-round PCR, nested PCR is performed using the first-round PCR product as a template. As primers, NUP primer contained in SMART PCR cDNA Synthesis Kit is used in combination with a specific forward primer or a specific reverse primer which is designed to bind internal sequence of the first-round PCR product of interest. PCR conditions are 1 cycle at 94˚C for 10 minutes; followed by 40 cycles of PCR, one cycle being 94˚C for 15 seconds, 63˚C for 15 seconds, and 72˚C for 2 minuets; and followed by 1 cycle at 72˚C for 7 minutes. The resulting PCR product is analyzed and purified by agarose gel electrophoresis to obtain DNA of interest. Further, the obtained DNA is cloned into the pCR-XL-TOPO vector (manufactured by Invitrogen), and sequenced.

The above sequencing results reveal 5'-terminal sequence and 3'-terminal sequence, each encoding N-terminal region and C-terminal region of the insect c-Jun $NH_2$-terminal kinase, respectively.

Thus, a polynucleotide shown in the polynucleotide group B can be obtained by PCR by preparing a primer based on an amino acid sequence of the known insect c-Jun $NH_2$-terminal kinase.

A polynucleotide having a nucleotide sequence complementary to a polynucleotide sequence of the polynucleotide group B can be used for obtaining a polynucleotide shown in the polynucleotide group B using a hybridization method.

The obtaining method in the present invention comprises a step of detecting a desired polynucleotide by hybridization, a step of identifying the detected desired polynucleotide, and a step of recovering the identified desired polynucleotide. Each step will be explained specifically below.

A step of detecting a desired polynucleotide by hybridization, and a step of identifying the detected desired polynucleotide can be performed by using, as a probe, a polynucleotide having a nucleotide sequence having complementarity to a nucleotide sequence of a polynucleotide group B, according to the method described, for example, in "Molecular Cloning: A Laboratory Manual 2nd edition" (1989), Cold Spring Harbor Laboratory Press, "Current Protocols In Molecular Biology" (1987), John Wiley & Sons,Inc.ISBNO-471-50338-X and the like.

Specifically, for example, a DNA having a nucleotide sequence having complementarity to a nucleotide sequence represented by SEQ ID NO: 2 is labeled with a radioisotope or a fluorescently labeled by the known method using Random Primed DNA Labelling Kit (manufactured by Boehringer) , Random Primer DNA Labelling Kit Ver. 2 (manufactured by TAKARA SHUZO Co., Ltd.), ECL Direct Nucleic Acid Labelling and Ditection System (manufactured by Amersham Biosciences), or Megaprime DNA-labelling system (manufactured by Amersham Biosciences),and this can be used as probe.

Examples of condition for hybridization include stringent condition, and specifically, examples include condition under which incubation is performed at 65˚C in the presence of 6xSSC (0.9M NaCl,0.09M sodium citrate), a 5×Denhart's solution (0.1%(w/v) Ficoll 400, 0.1%(w/v) polyvinylpyrrolidone, 0.1% BSA), 0.5%(w/v) SDS and 100 μg/ml denatured salmon spermatozoon DNA, or in a DIG EASY Hby solution (Boehringer Mamnnheim) containing 100 μg/ml denatured salmon spermatozoon DNA, then, incubation is performed two times at room temperature for 15 minutes in the presence of 1×SSC (0. 15 m NaCl, 0.015 m sodium citrate) and 0.5%SDFS and, further, incubation is performed at 68˚C for 30 minutes in the presence of 0.1×SSC (0.015 m NaCl, 0.0015 m sodium citrate) and 0.5%SDS. More specifically, for example, a probe labeled with $^{32}P$ can be made by employing a polynucleotide having a nucleotide sequence having complementarity to a nucleotide sequence of a polynucleotide group B as a template, using Megaprime DNA-labelling system (manufactured by Amersham Pharmacia Biotech) and using a reaction solution designated in a kit. Colony hybridization is performed using this probe according to a conventional method, incubation is performed at 65˚C in the presence of 6xSSC (0.9M NaCl,0.09M sodium citrate), a 5×Denhart's solution (0.1%(w/v) Ficoll 400, 0.1%(w/v) polyvinylpyrrolidone, 0.1%BSA), 0.5%(w/v) SDS and 100 μg/ml denatured salmon spermatozoon DNA, or in a DIG EASY Hyb solution (Boehringer Mannheim) containing 100 μg/ml denatured salmon spermatozoon DNA, then, incubation is performed two times at room temperature for 15 minutes in the presence of 1×SSC (0.15 m NaCl, 0.015 m sodium

citrate) and 0.5%SDS and, further, incubation is performed at 68°C for 30 minutes in the presence of 0.1×SSC (0.015 m NaCl, 0.0015 m sodium citrate) and 0.5%SDS, thereby, (a colony containing) a hybridizing polynucleotide can be detected. Thus, a desired polynucleotide can be detected by hybridization, and the detected desired polynucleotide can be identified.

For recovering the identified desired polynucleotide, a plasmid DNA can be recovered from a colony containing the polynucleotide detected and identified by the aforementioned method, for example, according to a method such as the alkali method described in "Molecular Cloning: A Laboratory Manual 2nd edition" (1989), Cold Spring Harbor Laboratory Press. A nucleotide sequence of the recovered desired polynucleotide (plasmid DNA) can be confirmed by a Maxam Gilbert method (described, for example, in Maxam,A.M & W.Gilbert, Proc.Natl.Acad.Sci.USA, 74, 560, 1977 etc.) or a Sanger method (described, for example, in Sanger,F. & A.R.Coulson, J.Mol.Biol., 94, 441, 1975,Sanger,F, & Nicklen and A.R.Coulson., Proc.Natl.Acad.Sci.USA, 74, 5463, 1977 etc.). Thereupon, for example, commercially available Termo Seqenase II dye terminator cycle sequencing kit (manufactured by Amersham biosciences), Dye Terminator Cycle Sequencing FS Ready Reaction Kit (manufactured by Applied Biosystems) and the like can be used.

A polynucleotide having a partial nucleotide sequence of a nucleotide sequence of the polynucleotide group B or a nucleotide sequence complementary to the partial nucleotide sequence can be used for obtaining a polynucleotide shown in the polynucleotide group B using PCR. More specifically, examples include a polynucleotide comprising a partial nucleotide sequence of a polynucleotide as described above or a nucleotide sequence complementary to said partial nucleotide sequence, which comprises a nucleotide sequence represented by SEQ ID NO: 3 or 4. The obtaining method in the present invention includes a step of amplifying a desired polynucleotide by PCR, a step of identifying the amplified desired polynucleotide, and a step of recovering the identified desired polynucleotide. Each step will be specifically explained below.

In a step of amplifying a desired polynucleotide by PCR, specifically, a DNA designed and synthesized from a partial nucleotide sequence of a nucleotide sequence of a polynucleotide group B or a nucleotide sequence having complementarity to the partial nucleotide sequence, based on an about 20bp to about 40bp nucleotide sequence, for example, a nucleotide sequence selected from a nucleotide sequence represented by SEQ ID NO: 2 and a sequence having complementarity to a nucleotide sequence represented by SEQ ID NO: 2 can be used as a primer set. Examples of a primer set include a set of a polynucleotide comprising a nucleotide sequence represented by SEQ ID NO: 3 and a polynucleotide comprising a nucleotide sequence represented by SEQ ID NO: 4. A PCR reaction solution is prepared, for example, by adding a reaction solution designated by a commercially available PCR kit to a cDNA library prepared by the aforementioned method. Reaction condition can be changed depending on a primer set to be used, and for example, condition under which after incubation at 94°C for 10 seconds, around 40 cycles is repeated, 1 cycle being 94°C for 15 seconds, 60°C for 15 seconds, and 72°C for 3 minutes and, further, incubation is performed at 72°C for 3 minutes, condition under which incubation is performed at 94°C for 2 minutes, thereafter, incubation is performed at about 8°C for 3 minutes and, thereafter, around 40 cycles is repeated, 1 cycle being 94°C for 30 seconds, 55°C for 30 seconds, and 72°C for 4 minutes, or condition under which 5 to 10 cycles is performed, 1 cycle being incubation at 94°C for 5 seconds and, then, 72°C for 4 minutes and, further, around 20 to 40 cycles is performed, 1 cycle being incubation at 94°C for 5 seconds and, then, 70°C for 4 minutes, can be used. In the PCR, for example, PfuUltra High Fidelity polymerase (manufactured by Stratagene), Amplitaq Gold (manufactured by Applied Biosystems), Takara Heraculase (Trademark) (manufactured by TAKARA SHUZO Co., Ltd.), a DNA polymerase contained in Advantage cDNA PCR Kit (manufactured by Clonetech), TaKaRa Ex Taq (manufactured by TAKARA SHUZO Co., Ltd.), PLATINUMTM PCR SUPER Mix (manufactured by Lifetech Oriental) can be used.

Identification of a desired polynucleotide amplified by PCR can be performed by measuring a molecular weight by agarose gel electrophoresis according to the method described in "Molecular Cloning: A Laboratory Manual 2nd edition" (1989), Cold Spring Harbor Laboratory Press. In addition, regarding the amplified desired polynucleotide, a sequencing reaction is performed using a commercially available DNA sequencing reaction kit, for example, Dye Terminator Cycle Sequencing FS Ready Reaction Kit (manufactured by Applied Biosystems) according to a manual annexed to the kit, and the nucleotide is analyzed using a DNA sequencer 3100 (manufactured by Applied Biosystems), thereby, a nucleotide sequence of the amplification fragment can be read.

Examples of a method of recovering the identified desired polynucleotide include a method of purifying and recovering the aforementioned polynucleotide identified by agarose gel electrophoresis from an agarose gel according to the method described in "Molecular Cloning: A Laboratory Manual 2nd edition" (1989), Cold Spring Harbor Laboratory Press. In addition, the thus recovered polynucleotide or a desired polynucleotide amplified by PCR can be cloned into a vector according to a conventional method described in "Molecular Cloning: A Laboratory Manual 2nd edition" (1989), Cold Spring Harbor Laboratory Press, and "Current Protocols In Molecular Biology" (1987), John Wiley & Sons,Inc. ISBNO-471-50338-X. Examples of a vector to be used include pUCA119 (manufactured by TAKARA SHUZO Co., Ltd.), pTVA118N (manufactured by TAKARA SHUZO Co., Ltd.), pBluescriptII (manufactured by Toyobo Co., Ltd.), pCR2.1-TOPO (manufactured by Invitrogen) and the like. In addition, a nucleotide sequence of the cloned polynucleotide can be confirmed by a Maxam Gilbert method (described, for example, in Maxam,A.M & W.Gilbert, Proc.Natl.Acad.Sci.USA,

74, 560, 1977) or a Sanger method (described, for example, in Sanger,F. & A.R.Coulson, J.Mol.Biol., 94, 441, 1975, Sanger,F, & Nicklen and A.R.Coulson., Proc.Natl.Acad.Sci.USA, 74, 5463, 1977). Thereupon, for example, a commercially available Termo Seqenase II dye terminator cycle sequencing kit (manufactured by Amersham biosciences), Dye Terminator Cycle Sequencing FS Ready Reaction Kit (manufactured by Applied Biosystems) and the like can be used.

In addition, a polynucleotide having a partial nucleotide sequence of a nucleotide sequence of the polynucleotide group B or a nucleotide sequence complementary to the partial nucleotide sequence can be used for obtaining a polynucleotide shown in the polynucleotide group B using not only a PCR method, but also the aforementioned hybridization method. More specifically, examples include a polynucleotide comprising a nucleotide sequence represented by SEQ ID NO: 3 or 4.

Examples of a method for preparing a protein having an amino acid sequence shown in the group B include a method of culturing a transformant with a polynucleotide selected from a polynucleotide group introduced therein, and recovering the produced protein. In addition, for preparing a transformant used herein, it is a work such as preparation of a circular polynucleotide containing a polynucleotide in which a polynucleotide selected from a polynucleotide group B is operably ligated to a baculovirus-derived promoter. The method will be explained in detail below.

In addition, a c-Jun $NH_2$-terminal kinase shown in a group A which is used in the method of assaying a pesticidal ability using a c-Jun $NH_2$-terminal kinase can be prepared and obtained by the similar method, using a polynucleotide having a nucleotide sequence encoding a c-Jun $NH_2$-terminal kinase used.

Baculovirus is a virus belonging to a diverse group of large double-stranded DNA viruses that infect many different species of insects as their natural hosts. The baculovirus genome is replicated and transcribed in the nuclei of infected host cells where the large circular baculovirus DNA (between 80 and 200 kb) is packaged into rod-shaped nucleocapsids. Examples of isolates used in foreign gene expression are Autographa californica multiple nuclear polyhedrosis virus (AcMNPV) and Bombyx mori (silkworm) nuclear polyhedrosis virus (BmNPV).

A baculovirus-derived promoter means a promoter of a gene contained in a baculovirus genome. Among them, examples of a promoter of baculovirus used for expressing a foreign gene include a promoter of a polyhedrin gene, and a promoter of a p10 gene (Harris and Polayes (1997). Focus 19, 6-8).

A promoter of a polyhedrin (polyhedron) gene is a promoter of a gene encoding polyhedrin which is a main component of an intranuclear inclusion body produced when baculovirus infects an insect cell. Polyhedrin is not a protein necessary for replicating a virus and, by substituting its gene with a gene of a protein of interest, the protein of interest amounting to 50% of a cell protein may be expressed.

In the present invention, "operably linked" means that a polynucleotide containing a gene of interest is linked downstream of a polynucleotide containing a promoter sequence so that the gene of interest can be transcribed in a used transcription system. Specifically, for example, when a promoter of a polyhedrin gene described later is used, a polynucleotide containing a gene of interest may be linked downstream of a promoter of a polyhedrin gene. In addition, for example, when a promoter other than a polyhedrin gene promoter is used, it is also possible to link a polynucleotide containing a gene of interest downstream of a polynucleotide containing a promoter sequence other than a polyhedrin gene promoter. More specifically, for example, when a plasmid pFastbacHT (manufactured by Invitrogen) vector utilizing a polyhedrin gene promoter is used, the polynucleotide can be operably linked by ligating a gene of interest into a restriction enzyme site such as BamHI, EcoRI, SalI, SpeI, NotI, XbaI, PstI, XhoI, SphI, KpnI, and HindIII located downstream of a polyhedrin gene promoter.

In the present invention, the "circular polynucleotide" is a polynucleotide which has been made to be circular by binding of ends of the polynucleotide strand, and examples include chromosomal DNAs of many bacteria in addition to a plasmid DNA, a bacmid DNA and the like.

A plasmid DNA is a relatively low-molecular circular polynucleotide, and examples include pET (manufactured by Takara Mirus Bio Inc.) and pBluescriptII (manufactured by Stratagene), used for cloning and expression in E. coli. Additional examples include pFastBac1, pFastBac HT A, pFastBac HT B, pFastBac HT C, pFastBac Dual, pBlueBacII (manufactured by Invitrogen), pAcSG2 (manufactured by Pharmingen) and the like, which contain a baculovirus expression cassette.

The bacmid is a high molecular weight DNA that consists of a BAC (bacterial artificial chromosome) that contains the entire baculoviral genome, for example bMON14272 (136 kb) that is present in DH10Bac™ *E. coli* cells (invitrogen). Bacmid DNA propagates as a large plasmid in *E. coli* cells and may contain an expression cassette for expression of a foreign gene under control of a baculoviral promoter.

A circular polynucleotide in which a polynucleotide comprising a nucleotide sequence encoding an amino acid sequence shown in the group B is operably linked to a baculovirus-derived promoter is specifically, for example, a circular polynucleotide containing a DNA comprising a cotton aphid c-Jun $NH_2$-terminal kinase gene operably linked to a polyhedrin promoter of baculovirus, and can be prepared and obtained, for example, according to the following method.

A plasmid DNA containing a cotton aphid c-Jun $NH_2$-terminal kinase gene cloned according to the aforementioned method is digested with EcoRI, and the resulting about 1.3kbp DNA fragment comprising a cotton aphid c-Jun $NH_2$-terminal kinase gene is ligated with a plasmid vector pFastBac HT B (manufactured by Invitrogen) which has been

digested with EcoRI in advance. The obtained plasmid is one example of a circular polynucleotide containing a DNA comprising a cotton aphid c-Jun $NH_2$-terminal kinase gene operably linked to a polyhedrin promoter of baculovirus. In addition, according to the manual annexed to Bac-to-Bac Baculovirus Expression System (manufactured by Invitrogen), this plasmid may be introduced into Escherichia coli DH10Bac, and a DNA containing a polyhedrin gene promoter and a cotton aphid c-Jun $NH_2$-terminal kinase gene can be inserted into a bacmid DNA by a recombination in the cell. For example, by the aforementioned method, a circular polynucleotide containing a DNA comprising a cotton aphid c-Jun $NH_2$-terminal kinase gene operably linked to a polyhedrin promoter of baculovirus can be prepared and obtained.

Similarly, a circular polynucleotide can be prepared by ligating a nucleotide encoding an amino acid sequence shown in the group B to a vector.

In the present invention, the "origin of replication" is the specific DNA sequence necessary for replicating itself in a host cell. Examples of origin of replication include colE1 and f1 for bacterial plasmids. In addition, a homologous repeated (hrs) region, and a non-hr region are present in a bacmid DNA (Pijlman et al. (2003) Journal of General Virology 84, 2669-2678).

One example of the circular polynucleotide is a baculovirus shuttle vector. Herein, a baculovirus shuttle vector means the bacmid DNA. The bacmid DNA can be propagated and genetically engineered in *E. coli*. Upon isolation from *E. coli* and introduction into an insect host cell, bacmids can be propagated as a virus. For example in the case of bMON14272 (invitrogen), the recombinant bacmids are generated in *E. coli* by transposition of a mini-Tn7 element, containing the baculoviral expression cassette from a pFastBac™ donor plasmid to the mini-attTn7 attachment site on the bacmid.

Insect cells to be used as host for the propagation of the baculovirus and for expression of the foreign protein by means of the recombinant baculovirus include cell lines derived from *Spodoptera frugiperda* or from *Trichoplusia ni.* Examples of such cell lines are, Sf21 cells, Sf9 cells, Tn-368 or High Five cells or Mimic Sf9 Insect cells (Invitrogen).

A transformant is a eukaryotic cell or a prokaryotic cell which has been genetically altered by introduction of a foreign polynucleotide into a cell. Examples of a transformant include an Escherichia coli cell transformed by introduction of a plasmid containing a baculovirus expression cassette such as plasmid vector pFastBac (manufactured by Invitrogen) and the like. In addition, examples of the technique of introducing a DNA into a host cell include transformations, transfection, protoplast fusion, lipofection, electroporation and the like.

Examples of a transformant in which a polynucleotide encoding an amino acid sequence shown in the group B is introduced include transformed Escherichia coli in which a DNA comprising a cotton aphid c-Jun $NH_2$-terminal kinase gene operably linked to a polyhedrin promoter of baculovirus is introduced. Specifically, the transformant can be prepared according to the following method.

A transformant can be prepared by introducing a plasmid vector pFastBac HT B (manufactured by Invitrogen) in which a DNA containing a cotton aphid c-Jun $NH_2$-terminal kinase gene is inserted in EcoRI site into an Escherichia coli cell according to the method described in "Molecular Cloning: A Laboratory Manual 2nd edition" (1989), Cold Spring Harbor Laboratory Press. In addition, a transformant can be also prepared by introducing the same plasmid vector into Escherichia coli DH10Bac according to a method described in a manual annexed to Bac-to-Bac Baculovirus Expression System (manufactured by Invitrogen).

Alternatively, a transformant can be also obtained by transfecting the bacmid DNA in which a fragment containing a polyhedrin gene promoter and a cotton aphid c-Jun $NH_2$-terminal kinase gene is inserted, into an insect cell according to a method described in a manual annexed to Bac-to-Bac Baculovirus Expression System (manufactured by Invitrogen).

A recombinant baculovirus is baculovirus in which the sequence of the baculovirus genome has been altered by genetic-engineering technique.

Specific examples of recombinant baculovirus include recombinant baculovirus containing a DNA fragment comprising a cotton aphid c-Jun $NH_2$-terminal kinase gene operably linked to a polyhedrin promoter of baculovirus. A recombinant baculovirus can be prepared, for example, by homologous recombination between baculoviral DNA and transfer vector DNA in insect cells (Kitts (1996) Cytotechnology 20, 111-123). Alternatively, recombinant baculovirus can be also prepared, for example, by introducing a recombinant bacmid DNA containing a DNA fragment comprising a cotton aphid c-Jun $NH_2$-terminal kinase gene operably linked to a polyhedrin promoter of baculovirus prepared by the aforementioned method into insect cells. Specifically, recombinant baculovirus can be prepared by transfecting a recombinant bacmid DNA containing a DNA fragment comprising a cotton aphid c-Jun $NH_2$-terminal kinase gene operably linked to the aforementioned polyhedrin promoter of Baculovirus into an insect cell according to the method described in a manual annexed to Bac-to-Bac Baculovirus Expression System (manufactured by Invitrogen). More specifically, the recombinant bacmid DNA is transfected into Sf9 cells ( ATCC : CRL-1711) according to the manual annexed to Bac-to-Bac Baculovirus (Trademark) Expression System (manufactured by Invitrogen) to obtain recombinant baculovirus. For the transfection, cellfectin (manufactured by Invitrogen), Grace's Insect Cell Culture Medium supplemented with L-amino acids (manufactured by Invitrogen, Gibco), 10% Foetal Bovine Serum (manufactured by Clontech), and penicillin/streptomycin (manufactured by Life Technologies) are used. In addition, for transfection, 2 $\mu$g of bacmid DNA and 7 $\mu$l of cellfectin are used. A P1 recombinant baculovirus stock is recovered after 8 days according to the manual annexed

to Bac-to-Bac Baculovirus (Trademark) Expression System (manufactured by Invitrogen). For example, 0.5 ml of this baculovirus stock can be further propagated by inoculating on 300 ml of Sf9 cell cultures of $1x10^6$ cells/ml. The amplified baculovirus stock is harvested after 4 days according to the manual annexed to Bac-to-Bac Baculovirus (Trademark) Expression System (manufactured by Invitrogen). The Sf9 cell are suspension-cultured in Erlenmeyer flasks at 27˚C and at 135 rpm. A component of a medium used in this culturing include Grace's Insect Cell Culture Medium supplemented with L-amino acids (manufactured by Invitrogen, Gibco), 10% Foetal Bovine Serum (manufactured by Clontech), penicillin/streptomycin (manufactured by Life Technologies), and final consideration 0.1% Pluronic F-68(manufactured by Sigma-aldrich).

A c-Jun $NH_2$-terminal kinase can be prepared by culturing a transformant prepared by the aforementioned method, and recovering the produced insect-derived c-Jun $NH_2$-terminal kinase.

C-Jun $NH_2$-terminal kinase protein may be produced by for example a recombinant baculovirus/Sf9 cell expression system. This system is one of the most powerful and versatile eukaryotic expression systems available, and may be used to express heterologous genes from many different sources, including fungi, plants, bacteria and viruses.

Alternatively, c-Jun $NH_2$-terminal kinase protein may be produced by a recombinant *E. coli* expression system. This system is the most frequently used prokaryotic expression system for the high-level production of heterologous proteins. *E. coli* is genetically and physiological the best characterized organism known, it is easy to manipulate, many tools are available, it is able to grow very fast, it grows on cheap complex or well-defined minimal media and it has an extremely high capacity to synthetize heterologous protein.

In addition, an insect-derived c-Jun $NH_2$-terminal kinase produced by culturing a transformant is lysed by a method such as sonication, French press, and Dyno mill, and recovered in a form contained in a cell crude extract, and a purified protein can be obtained by using a procedure conventionally used in enzyme purification such as ion exchange column chromatography, reverse phase column chromatography, gel filtration column chromatography and the like. Alternatively, when it is devised that an insect-derived c-Jun $NH_2$-terminal kinase is produced in a form with His-tag, a purified protein can be obtained rapidly from a cell crude extract by affinity column chromatography which specifically recognizes and binds to the His-tag. By the method, an insect-derived c-Jun $NH_2$-terminal kinase can be prepared.

For example, an insect-derived c-Jun $NH_2$-terminal kinase can be prepared by culturing a transformed insect cell with a DNA fragment containing a cotton aphid c-Jun $NH_2$-terminal kinase gene operably linked to a polyhedrin promoter of baculovirus, and grinding the cell with a French press, followed by purification with column chromatography.

More specifically, for example, $4\times10^8$ Sf9 cells are suspended in 30 ml of a stock solution of recombinant baculovirus containing a recombinant bacmid DNA containing a DNA fragment comprising a cotton aphid c-Jun $NH_2$-terminal kinase gene operably ligated to a polyhedrin promoter of baculovirus, the suspension is rotation-cultured in 125 ml Erlenmeyer at 135rpm, at 27˚C for 1 hour. Thereafter, each 1/3 of the cell suspension is placed into three 25 ml Erlenmeyer flasks, a medium is added to each flask so that a volume of a culturing solution becomes 100 ml, 1 ml of a 10% Pluronic solution is added, and this is cultured again. After 48 hours, Sf9 cells infected with baculovirus are harvested by centrifugation at 290xg for 5 minutes. Buffer A (50 mM Hepes-HaOH pH7.5, 0.5 m NaCl, 10 mM imidazole)is added to the harvested Sf9 cells to suspend them, and cells are lysed at a pressure of 1,500psi using a French press (manufactured by Thermo Spectronic) according to a method described in the annexed manual. This French-pressed solution is centrifuged at 13,000mg at 4˚C for 20 minutes, and the resulting supernatant is filtered through a 0.45 mm filter. Then, this is injected into two HiTrap/HisTrap affinity columns (column volume 5 ml, manufactured by Amersham biosciences) connected in series which are equilibrated with buffer A (50 mM Hepes-HaOH pH7.5, 0.5 m NaCl, 10 mM imidazole), and columns are washed with 100 ml of buffer A. Then, columns are washed with 150 ml of a buffer obtained by mixing 93% of buffer A and 7% of buffer B (50 mM Hepes-HaOH pH7.5, 0.5 m NaCl, 500 mM imidazole). Finally, 60 ml of a buffer obtained by mixing 50% of buffer A and 50% of buffer B is injected into columns. Each 1 ml of the eluted fractions are fractionated, and stored, and an aliquot is analyzed with SDS-PAGE to identify fractions containing 46Kda of a c-Jun $NH_2$-terminal kinase.

Further, to increase the activity of c-Jun $NH_2$-terminal kinase, the c-Jun $NH_2$-terminal kinase may be incubated with MKK4/SKK1, active (manufacture by Upstate)and MKK7 beta 1, active (manufactured by Upstate). In this case, a part of kinase assay methods described in manuals annexed to MKK4/SKK1, active (manufactured by Upstate) and MKK7 beta 1, active (manufactured by Upstate) may be modified, and a c-Jun $NH_2$-terminal kinase can be activated. The required buffers and reagents include:

(i) Hepes buffer, $MgCl_2$, ATP;
(ii) Recombinant MKK4/SKK1, active expressed in Escherichia coli (Upstate) stocked at a final concentration of 53.5$\mu$M in 50 mM Tris/HCl, pH 7.5, 150 mM NaCl, 0.1 mM EGTA, 0.03% Brij 35, 270 mM sucrose, 1 mM benzamidine, 0.2 mM PMSF, 0.1% 2-mercaptoethanol; and
(iii) Recombinant MKK7 beta 1, active expressed in Escherichia coli (Upstate) stocked at a final concentration of 14.6$\mu$M in 50 mM Tris/HCl, pH 7.5, 150 mM NaCl, 0.1 mM EGTA, 0.03% Brij 35, 270 mM sucrose, 1 mM benzamidine, 0.2 mM PMSF, 0.1% 2-mercaptoethanol.

Phosphorylation of a purified c-Jun $NH_2$-terminal kinase is performed at a final volume of 10 ml by the following procedure.

First, all of 1 ml of Milli Q water, 1ml of a 500 mM Hepes buffer, 2 ml of a mixture of MKK4/SKK1, active (375 nM) and MKK7 betal, active (375 nM), and 2ml of a mixture of $MgCl_2$ (75 mM) and ATP (570 $\mu$M) are mixed, and 4ml of c-Jun $NH_2$-terminal kinase (1 mg/ml) is added to the mixture. Then, the mixture is incubated at room temperature for 1 hour. All c-Jun $NH_2$-terminal kinases which have been activated with different batches are pooled, and glycerol is added to this to a final concentration of 10%. Then, this is divided into each 1 ml, and stored at -80˚C.

It is to be understood that other methods of activation are also possible, for example: in vivo stimulation as described by Ian N. Foltz, et al. (The Journal of Biological Chemistry (1998) Vol 273, 9344-9351).

An insect c-Jun $NH_2$-terminal kinase comprising an amino acid sequence shown in the group B can be used as a research tool. For example, it can be used as a research tool for performing study such as assaying of the pestcidal ability, screening of a chemical substance having a pestcidal ability, and the like. In addition, for example, also in study of analyzing action and mechanism of an agent which acts on a c-Jun $NH_2$-terminal kinase, a c-Jun $NH_2$-terminal kinase can be utilized as a research tool.

In addition, polynucleotides encoding amino acid sequences shown in the group B and polynucleotides having a nucleotide sequence having complementarity to them, as well as partial nucleotide sequences of polynucleotides encoding amino acid sequences shown in the group B, or polynucleotides having nucleotide sequences having complementarity to the partial nucleotide sequences, and a polynucleotide complying a nucleotide sequence represented by SEQ ID NO: 3 or 4 can be used as a research tool. For example, a part of them functions as a polynucleotide used in a method of preparing a c-Jun $NH_2$-terminal kinase as described above. In addition, a part can be used as an important research tool for performing obtaining a polynucleotide shown in a polynucleotide group B using PCR, or obtaining a polynucleotide shown in a polynucleotide group B using hybridization, as described above.

Particularly, upon implementation of screening of a pestcidal agent, they can be used as an experimental tool for an experiment which is performed for screening. Specifically, the herein described c-Jun-$NH_2$-terminal kinase or the herein disclosed polynucleotide can be used as an experimental tool for an experiment which is performed upon implementation of the assaying of a pestcidal ability, screening of a chemical substance having a pestcidal ability, and the like.

Further, also described herein is a system which comprises a means to input, store and manage data information of an ability of test substances, wherein said ability is an ability to modulate the activity of an insect c-Jun $NH_2$-terminal kinase (hereinafter, referred to as means a in some cases), a means to query and retrieve the data information based on a desired criterion (hereinafter, referred to as means b in some cases), and a means to display and output the result which is queried and retrieved (hereinafter, referred to as means c in some cases) (hereinafter, referred to as present system in some cases).

First, a means a will be explained. A means a is a means to, after data information of an ability to modulate the activity of an insect-derived c-Jun $NH_2$-terminal kinase possessed by the test substance is inputted, store and manage the inputted information, as described above. The information is inputted by an inputting means 1, and is usually memorized in a memory means 2. Examples of an inputting means include means which can input the information such as a keyboard and a mouse. When inputting and storing managing of the information are completed, a procedure progresses to a next means b. For storing managing the information, a large amount of data may be effectively stored and managed by inputting information having a data structure using a hardware such as a computer, and a software such as OS and database management, and storing the information into a suitable memory device, for example, computer-readable recording medium such as a flexible disc, a photomagnetic disc, CD-ROM, DVD-ROM, and a hard disc.

A means b will be explained. A means b is a means to query and retrieve the data information stored and managed by a means of a based on criterion for obtaining a desired result, as described above. For the information, when criterion for querying and retrieving is inputted by an inputting means 1, and information in conformity with the criterion is selected among the information usually memorized in a memory means 2, a procedure progresses to a next means c. The selected result is usually memorized in a memory means 2 and, further, can be displayed by a displaying outputting means 3.

A means c will be explained. A means c is a means to display and output the result which is queried and retrieved, as described above. Examples of the displaying outputting means 3 include a display, a printer and the like, and the result may be displayed on a display device of a computer, or may be outputted on a paper by printing.

EXAMPLES

The present invention will be explained in more detail below by way of Examples, but the present invention is not limited to these particular Examples.

Example 1 (Extraction of total RNA from cotton aphid and German cockroach)

(1) Extraction of total RNA from cotton aphid.

A population of adults and larvae of cotton aphid (Aphis *gossypii),* which had been reared on leaves of potted cucumber, was scraped from the surface of the leaves with a small brush, and 630 mg of the obtained population was crushed into a powder in liquid nitrogen using a mortar and a pestle. From the resulting frozen crushed powder, RNA was isolated using a RNA extracting reagent ISOGEN (manufactured by Nippon Gene) as follows. After 10 ml of ISOGEN was added to the frozen crushed powder in the mortar, the crushed powder was ground for 10 minutes while kept on ice. After grinding, a fluid sample was transferred to a 15 ml tube with a pipette, and 2ml of chloroform (manufactured by Wako Pure Chemical Industries, Ltd.) was added thereto. Immediately, the mixture was vigorously shaken for 15 seconds and then left at rest at room temperature for 3 minutes. Then, the resulting mixture was centrifuged at 12,000×g at 4˚C for 15 minutes, and each 5 ml of aqueous layer were transferred to two new tubes. After 5 ml of ISOGEN was added to each tube, the mixture was immediately shaken vigorously for 15 seconds, and left at rest at room temperature for 3 minutes. Then, the resulting mixture was centrifuged at 12,000×g at 4˚C for 15 minutes, and each 10 ml of aqueous layer were transferred to new 50 ml tubes, respectively. Subsequently, 10 ml of isopropanol (manufactured by Wako Pure Chemical Industries, Ltd.) was added to each tube, and the mixture was kept on ice for 30 minutes. The resulting mixture was centrifuged at 12,000×g at 4˚C for 10 minutes to precipitate RNA. After the supernatant was removed, 20 ml of 70% ethanol was added to the residue. The resulting mixture was centrifuged at 10,000xg at 4˚C for 5 minutes. After the supernatant was removed, the precipitate of total RNA was slightly dried and then dissolved in 1 ml of commercially available RNase-free water (Nacalai Tesque, Inc.). A concentration of the prepared total RNA (calculated from an absorbance at 260 nm) was 6.9mg/ml.

(2) Extraction of total RNA from German cockroach

Adults, nymphs and oothecae of artificially-reared German cockroach (Blattella *germanica)were* provided as samples. Ten (10) of adult males and 10 of adult females (individuals from each of which ootheca has been removed) were used as an adult sample of 1.1g, 10 of nymph males and 10 of nymph females were used as a nymph sample of 1.0g, and 26 oothecae were used as an ootheca sample of 1.0g. Three kinds of these samples were separately crushed into a powder in liquid nitrogen using separate mortars and pestles. From each of the resulting frozen crushed powders, RNA was isolated using a RNA extracting reagent ISOGEN (manufactured by Nippon Gene) as follows. After 10 ml of ISOGEN was added to the frozen crushed powder in the mortar, the crushed powder was ground for 10 minutes while kept on ice. After grinding, a fluid sample was transferred to a 15 ml tube with a pipette, and 2ml of chloroform (manufactured by Wako Pure Chemical Industries, Ltd.) was added thereto. Immediately, the mixture was vigorously shaken for 15 seconds and then left at rest at room temperature for 3 minutes. Then, the resulting mixture was centrifuged at 12,000×g at 4˚C for 15 minutes, and each 5 ml of aqueous layer were transferred to two new tubes. After 5 ml of ISOGEN was added to each tube, the mixture was immediately shaken vigorously for 15 seconds, and left at rest at room temperature for 3 minutes. Then, the resulting mixture was centrifuged at 12,000xg at 4˚C for 15 minutes, and each 10 ml of aqueous layer were transferred to new 50 ml tubes, respectively. Subsequently, 10 ml of isopropanol (manufactured by Wako Pure Chemical Industries, Ltd.) was added to each tube, and the mixture was kept on ice for 30 minutes. The resulting mixture was centrifuged at 12,000xg at 4˚C for 10 minutes to precipitate RNA. After the supernatant was removed, 20 ml of 70% ethanol was added to the residue. The resulting mixture was centrifuged at 10,000xg at 4˚C for 5 minutes. After the supernatant was removed, the precipitate of total RNA was slightly dried and then dissolved in 1 ml of commercially available RNase-free water (Nacalai Tesque, Inc.). A concentration of the prepared total RNA (calculated from absorbance at 260 nm) was 1.1 mg/ml in the case of adult-derived total RNA was 2.5 mg/ml in the case of nymph-derived total RNA, and 1.4 mg/ml in the case of ootheca-derived total RNA.

Example 2 (Isolation of cotton aphid c-Jun $NH_2$-terminal kinase gene)

First-strand cDNA was prepared using total RNA from cotton aphid, random Primers (Invitrogen) and Superscript III (Invitrogen) for RT-PCR according to the manufacturer's procedure of Superscript III.

A full-length cDNA of cotton aphid c-Jun $NH_2$-terminal kinase was amplified by PCR using an oligonucleotide comprising the nucleotide sequence of SEQ ID NO: 3 and an oligonucleotide comprising the nucleotide sequence of SEQ ID NO: 4, which are primers specific for the gene, and Pfu Turbo (manufactured by Stratagene) according to the manufacturer's procedure. First-strand cDNA, prepared as described above, was used as template. The PCR conditions used were as follows: an initial denaturation at 94˚C for 10 minutes; followed by 30 cycles of PCR, one cycle being 94˚C for 30 seconds, 50˚C for 30 seconds, and 72˚C for 2 minutes; followed by 72˚C for 7 minutes. The resulting PCR products were analyzed and purified by agarose gel electrophoresis to obtain the 1260bp DNA of interest. The obtained DNA

was cloned into the pCR-blunt vector (Invitrogen). The resulting plasmid was named oGA058-59 in pCRblunt. The cloned DNA was sequenced to confirm that a polynucleotide comprising the nucleotide sequence of SEQ ID No: 2 was contained in the cloned DNA. An amino acid sequence presumed from the nucleotide sequence was the amino acid sequence of SEQ ID No: 1.

Example 3 (Isolation of German cockroach c-Jun $NH_2$-terminal kinase Gene)

For isolation of homologues of c-Jun $NH_2$-terminal kinase gene from other insect species such as German cockroach (Blatella germanica), degenerate primers are designed using Codehop program (publicly accessible on the website of Blocks Protein Analysis Server operated within the Fred Hutchinson Cancer Research Center at http://blocks.fhcrc.org/blocks/codehop.html), and based on the sequence of the aforementioned cotton aphid-derived c-Jun $NH_2$-terminal kinase gene and the previously-known nucleotide sequences of Ancylostoma caninum (NCBI accession number AAF00539), Xenopus laevis (BAB91438, BAB85483), Homo sapiens (NP_620448, NP_002744), Mus musculus (BAC31240), Rattus norvegicus (S43969), Gallus gallus (BAA19188), Drosophila melanogaster (AAF52883, AAC47325), Anopheles gambiae (EAA05905) and Aedes albopictus(AA031950).

Partial sequences of a homologue of c-Jun $NH_2$-terminal kinase gene in a selected insect species are amplified by a series of PCR using first-strand cDNA derived from the insect species as a template. Herein, the first-strand cDNA as a template is prepared by the aforementioned method using Superscript III. Amplification by PCR is performed using a set of degenerate primers as a forward primer and a reverse primer as well as Amplitaq Gold (manufactured by Applied Biosystems) according to the manufacturer's procedure annexed to the reagent. PCR conditions are 94˚C for 10 minutes; followed by 40 cycles of PCR, one cycle being 94˚C for 30 seconds, 45˚C for 1 minute, and 72˚C for 1 minute per 1kb of a length of a predicted amplification product; and followed by 72˚C for 7 minutes. The PCR product is analyzed and purified by agarose gel electrophoresis to obtain DNA of interest. Further, the obtained DNA is cloned into the pCR-XL-TOPO vector (manufactured by Invitrogen), and sequenced.

Thus, partial sequence of a c-Jun $NH_2$-terminal kinase gene of Blatella germanica comprising the nucleotide sequence of SEQ ID NO: 15 was obtained. Amino acid sequence presumed from the partial sequences was the amino acid sequence of SEQ ID NO: 16.

Then, primers specific for the resulting partial sequences of the insect homologue of c-Jun $NH_2$-terminal kinase gene are designed, and 3'RACE PCR or 5'RACE PCR is performed in order to obtain a full-length sequence of the gene. 3'and 5' RACE PCRs are performed employing first-strand cDNA prepared from the insect total RNA as a template and using SMART PCR cDNA Synthesis Kit (manufactured by Clontech) according to the manufacturer's instructions annexed to the kit.

In 3'RACE and 5'RACE reactions, universal primer mix (UPM) contained in SMART PCR cDNA Synthesis Kit is used in combination with a forward primer or a reverse primer which is specific for the sequence of interest. PCR conditions are 1 cycle at 94˚C for 10 minutes; followed by 40 cycles of PCR, one cycle being 94˚C for 15 seconds, 63˚C for 15 seconds, and 72˚C for 1 minute per 1kb of a length of a predicted amplification product; and followed by 1 cycle at 72˚C for 7 minutes. The resulting PCR product is analyzed and purified by agarose gel electrophoresis to obtain DNA of interest. Further, the obtained DNA is cloned into the pCR-XL-TOPO vector (manufactured by Invitrogen), and sequenced.

When a distinct amplification product is not obtained by the first-round PCR, nested PCR is performed using the first-round PCR product as a template. As primers, NUP primer contained in SMART PCR cDNA Synthesis Kit is used in combination with a specific forward primer or a specific reverse primer which is designed to bind internal sequence of the first-round PCR product of interest. PCR conditions are 1 cycle at 94˚C for 10 minutes; followed by 40 cycles of PCR, one cycle being 94˚C for 15 seconds, 63˚C for 15 seconds, and 72˚C for 2 minuets; and followed by 1 cycle at 72˚C for 7 minutes. The resulting PCR product is analyzed and purified by agarose gel electrophoresis to obtain DNA of interest. Further, the obtained DNA is cloned into the pCR-XL-TOPO vector (manufactured by Invitrogen), and sequenced.

The above sequencing results reveal 5'-terminal sequence and 3'-terminal sequence, each encoding N-terminal region and C-terminal region of the insect c-Jun $NH_2$-terminal kinase, respectively.

Example 4 (Construction of recombinant bacmid)

(1) Cloning of c-Jun $NH_2$-terminal kinase cDNA into gene expression vector pFastBac (Registered Trademark) HTb

A 1272bp DNA fragment, obtained by digesting with EcoRI oGA058-59 in pCRblunt that had been obtained in Example 2, was isolated and purified, and ligated into the EcoRI cloning sites of the gene expression vector pFastBac (Registered Trademark) HTb. Hereinafter, the resulting vector was named oGA058-59 in pFastBacHTb.

Then, PCR was performed to obtain a 663 bp DNA fragment using Pfu Turbo polymerase (Stratagene) and

employing oGA058-59 in pCRblunt obtained in Example 2 as a template. The PCR was performed according to the manual annexed to the reagent and, as primers, an oligonucleotide comprising the nucleotide sequence of SEQ ID NO: 5 and an oligonucleotide comprising the nucleotide sequence of SEQ ID NO: 6, which are primers having high specificity, were used. The PCR conditions were as follows: 1 cycle of 95˚C for 5 minutes; followed by 25 cycles of PCR, one cycle being 95˚C for 30 seconds, 50˚C for 30 seconds and 72˚C for 1 minute and 30 seconds; and followed by 1 cycle of 72˚C for 7 minutes. Then, a DNA fragment of interest was obtained by analyzing and purifying the resulting PCR product by agarose gel electrophoresis. Further, the obtained DNA fragment was cloned into pCR-blunt vector (manufactured by Invitrogen), and a nucleotide sequence of the DNA fragment was determined. The thus obtained vector was designated as oGA060-61 in pCRblunt.

A 605 bp DNA fragment obtained by digesting the above-obtained oGA060-61 in pCRblunt with BamHI/NdeI was isolated and purified, and ligated to the BamHI/NdeI cloning site of oGA058-59 in pFastBacHTb. The resulting vector was designated as pGA01.1. Then, a nucleotide sequence was determined and, as a result, it was found out that a mutation was contained in pFastBacHTb used in construction.

A digestion product obtained by digesting pGA01.1 with BamHI and EcoRI was analyzed and purified by agarose gel electrophoresis to isolate and purify a 1241 bp DNA fragment of interest, and the DNA fragment was cloned into a 4850 bp BamHI/EcoRI fragment of the expression vector pFastBac (Registered Trademark) HTb. The resulting vector was designated pGAO1 and replaced the former pGAO1.1 construct.

(2) Generation of recombinant bacmid DNA

Using the obtained pGAO1, a competent cell of *Escherichia coli* DH10Bac was transformed. Recombinant bacmid DNA for cotton aphid c-Jun $NH_2$-terminal kinase was isolated from the transformed Escherichia coli DH10Bac. All procedures were according to the manual annexed to Bac-to-Bac Baculovirus (Trademark) Expression System (manufactured by Invitrogen).

The presence or the absence of the objective gene in the recombinant bacmid was verified by PCR analysis. As the bacmid contains M13 Forward(-40) and M13 Reverse priming sites, the M13Forward(-40) and the M13Reverse primers were used. Also a combination the M13 Forward(-40) or M13 Reverse primers and a primer specific for the insert was also used. Each manipulation was performed according to the manual annexed to Bac-to-Bac Baculovirus (Trademark) Expression System (manufactured by Invitrogen). Each PCR condition was as follows.

(a) In the case of an oligonucleotide comprising the nucleotide sequence of SEQ ID NO: 7 and an oligonucleotide comprising the nucleotide sequence of SEQ ID NO: 8:

(i) 94˚C for 5 minutes; followed by
(ii) 40 cycles of 94˚C for 15 seconds, 60˚C for 15 seconds, and 72˚C for 3 minutes; and followed by
(iv) 72˚C for 7 minutes.

(b) In the case of an oligonucleotide comprising the nucleotide sequence of SEQ ID NO: 9 and an oligonucleotide comprising the nucleotide sequence of SEQ ID NO: 10:

(i) 94˚C for 5 minutes; followed by
(ii) 16 cycles of 94˚C for 15 seconds, 68˚C (reduction of 0.5˚C every +1 cycle) for 15 seconds, and 72˚C for 3 minutes; followed by
(iii) 20 cycles of 94˚C for 15 seconds, 60˚C for 15 seconds, and 72˚C for 3 minutes; and followed by
(iv) 72˚C, 7 minutes.

(c) In the case of an oligonucleotide comprising the nucleotide sequence of SEQ ID NO: 11 and an oligonucleotide comprising the nucleotide sequence of SEQ ID NO: 12:

(i) 94˚C for 5 minutes; followed by
(ii) 40 cycles of 94˚C for 15 seconds, 60˚C for 15 seconds, and 72˚C for 3 minutes; and followed by
(iv) 72˚C for 7 minutes.

(d) In the case of an oligonucleotide comprising the nucleotide sequence of SEQ ID NO: 8 and an oligonucleotide comprising the nucleotide sequence of SEQ ID NO: 9:

(i) 94˚C for 5 minutes; followed by
(ii) 20 cycles of 94˚C for 15 seconds, 65˚C for 15 seconds, and 72˚C for 3 minutes; followed by

(iii) 25 cycles of 94˚C for 15 seconds, 65˚C for 15 seconds, and 72˚C for 3 minutes (increase of 5 seconds every +1 cycle); and followed by
(iv) 72˚C, 7 minutes.

Based on the size of the amplified DNA fragments, two kinds of recombinant bacmids of pgaol.1 and pgaol.8 were selected. DNA fragments amplified from both bacmids had the correct size. Therefore, it was indicated that transposition of the pFastBac (Registered Trademark) expression construct into the bacmid DNA had occurred.

Example 5 (Preparation of recombinant bacmid stock)

(1) Transfection of recombinant bacmid

The recombinant bacmid pgaol.1 was transfected into Sf9 cells (ATCC: CRL-1711) in order to generate recombinant baculovirus according to the manual annexed to Bac-to-Bac Baculovirus (Trademark) Expression System (manufactured by Invitrogen). For the transfection, following materials were used: cellfectin (manufactured by Invitrogen), Grace's Insect Cell Culture Medium supplemented with L-amino acids (manufactured by Invitrogen, Gibco), 10% of Foetal Bovine Serum (manufactured by Clontech), and penicillin/streptomycin (manufactured by Life Technologies). 2 μg of bacmid DNA and 7 μl of cellfectin were used. P1 recombinant baculovirus stock was harvested after 8 days according to the manual annexed to Bac-to-Bac Baculovirus (Trademark) Expression System (manufactured by Invitrogen).

<Large scale preparation of recombinant baculovirus>

The cotton aphid c-Jun $NH_2$-terminal kinase baculovirus stock was amplified by inoculating 0.5 ml of P1 virus stock or subsequent generated virus stock (MOI=0.1) on 300 ml of Sf9 insect cell cultures of $1\times10^6$ cells/ml. The amplified baculovirus stocks were harvested after 4 days of culture according to the manual annexed to Bac-to-Bac Baculovirus (Trademark) Expression System (manufactured by Invitrogen). The Sf9 cell cultures were grown as suspension in Erlenmeyers (Elscolab) at 135 rpm and at 27˚C. The medium components were as follows: Grace's Insect Cell Culture Medium supplemented with L-amino acids (manufactured by Invitrogen, Gibco), 10% Foetal Bovine Serum (manufactured by Clontech), penicillin/streptomycin (manufactured by Life Technologies), and pluronic F-68 solution at final concentration of 0.1 % (manufactured by Sigma-aldrich).

The titer of the baculovirus stock was determined by plaque assay according to the manual annexed to Bac-to-Bac Baculovirus (Trademark) Expression System (manufactured by Invitrogen), but for the medium of the plaque assay, 2% agarose was used instead of 4% agarose.

Example 6 (Expression of c-Jun $NH_2$-terminal kinase in insect cell)

Sf9 cells of $4\times10^8$ were suspended in 30 ml of recombinant baculovirus stock in which the cotton aphid c-Jun $NH_2$-terminal kinase gene was introduced, and rotation-cultured in a 125 ml Erlenmeyer (manufactured by Elscolab) at 27˚ C, 135 rpm for 1 hour. Thereafter, the cell suspension was equally distributed over three 250 ml Erlenmeyer (manufactured by Elscolab), and the cell culture medium described in Example 5 was added until a volume of a culturing solution in each Erlenmeyer became 100 ml. The Sf9 cells were rotation-cultured at 135rpm at 27˚C for 48 hours in the prepared culturing solution. The cultured solution was centrifuged at 1200rpm to harvest Sf9 cells infected with baculovirus. The harvested Sf9 cells were flash frozen in liquid nitrogen and stored at -80˚C until further use.

Example 7 (Purification of c-Jun $NH_2$-terminal kinase)

(1) Preparation of crude extract

The frozen Sf9 cells infected with baculovirus were re-suspended in 30 ml of buffer A (50 mM Hepes pH 7.5, 0.5 m NaCl, 10 mM imidazole), and subsequently lysed in buffer A by means of a French Press (manufactured by Thermo Spectronic). The pressure was maintained at 1300-1500 psi during the procedure of breaking of the cells. The French pressed solution was centrifuged at 13,000×g at 2˚C for 20 minutes to obtain supernatant. The obtained supernatant was filtered through a 0.45μm filter and kept on ice.

(2) Purification

The aphid c-Jun $NH_2$-terminal kinase was cloned in frame with the 6xHis tag in pFastBacHTb. The recombinant

protein has been purified using metal affinity chromatography, using either the HiTrap Chelating HP (Amersham biosciences) or HisTrap HP (Amersham biosciences) columns, according to the instructions of the manufacturer (Amersham biosciences). The purification procedure was undertaken on the ÄKTA-FPLC (Amersham biosciences).

The HiTrap/HisTrap affininity columns have been prepared according to the manufacturer's protocol (Amersham biosciences). Buffer A, the binding buffer, was made of 50mM Hepes pH 7.5, 0.5M NaCl, 10 mM imidazole; and buffer B, the elution buffer, was made of 50mM Hepes pH 7.5, 0.5M NaCl, 500mM Imidazole. The purification protocol to purify the aphid c-Jun $NH_2$-terminal kinase included the following steps:

(i) sample injection,
(iI) washing out unbound protein with 10 column volumes (CV) of buffer A,
(111) washing for 15 CV with 15% buffer B (75 mM imidazole) for Equilibration of the column,
(iv) elution of purified protein with 13 CV 50 % buffer B (250 mM imidazole), and
(v) washing the column with 10 CV 100 % buffer B (500 mM imidazole).

The obtained elution fractions were analysed to verify presence of the recombinant cotton aphid peptidy-dipeptidase A by means of standard techniques of SDS-PAGE and western blot. An 8% polyacrylamide gel was used for optimal gel electrophoresis resolution of the c-Jun $NH_2$-terminal kinase protein, since the molecular weight of the c-Jun $NH_2$-terminal kinase protein was 46kDa. For western blot analysis, an anti - His(H15) sc-803 rabbit polyclonal IgG antibody (manufactured by tebubio) was used as primary antibody at a 1:500 dilution. The secondary antibody was a goat anti-rabbit-HRP (manufactured by Pierce) at a dilution of 1:10000.

After analysis of the gels by SDS-PAGE and Western blotting, the fractions of interest were pooled and glycerol was added to a final concentration of 10%. The protein concentration was determined with the Bradford spectro-photometric protocol using Pre-diluted Protein Assay Standards: Bovine Serum Albumin Fraction V Set according to the manufacturer's protocol (Bio-Rad). The pooled fractions were then distributed into several aliquots and immediately flash-frozen in liquid nitrogen and stored at -80 ˚C.

(3) Activation of c-Jun $NH_2$-terminal kinase

To increase the activity of c-Jun $NH_2$-terminal kinase, the c-Jun $NH_2$-terminal kinase was incubated with MKK4/SKK1, active (manufactured by Upstate) and MKK7 beta 1, active (manufactured by Upstate). In this case, a part of kinase assay methods described in manuals annexed to MKK4/SKK1, active (manufactured by Upstate) and MKK7 beta 1, active (manufactured by Upstate) was modified, and a c-Jun $NH_2$-terminal kinase was activated.

The required buffers and reagents were as follows:

(i) Hepes buffer, $MgCl_2$, ATP;
(ii) Recombinant MKK4/SKK1, active expressed in Escherichia coli (Upstate) stocked at a final concentration of 53.5$\mu$M in 50 mM Tris/HCl, pH 7.5, 150 mM NaCl, 0.1 mM EGTA, 0.03% Brij 35, 270 mM sucrose, 1 mM benzamidine, 0.2 mM PMSF, 0. 1% 2-mercaptoethanol; and
(iii) Recombinant MKK7 beta 1, active expressed in Escherichia coli (Upstate) stocked at a final concentration of 14.6$\mu$M in 50 mM Tris/HCl, pH 7.5, 150 mM NaCl, 0.1 mM EGTA, 0.03% Brij 35, 270 mM sucrose, 1 mM benzamidine, 0.2 mM PMSF, 0.1% 2-mercaptoethanol.

Phosphorylation of a purified c-Jun $NH_2$-terminal kinase was performed at a final volume of 10 ml by the following procedure.

First, all of 1 ml of Milli Q water, 1ml of a 500 mM Hepes buffer, 2 ml of a mixture of MKK4/SKK1, active (375 nM) and MKK7 betal, active (375 nM), and 2ml of a mixture of $MgCl_2$ (75 mM) and ATP(570 $\mu$M) were mixed, and 4ml of c-Jun $NH_2$-terminal kinase (1 mg/ml) was added to the mixture. Then, the mixture was incubated at room temperature for 1 hour. All c-Jun $NH_2$-terminal kinases which had been activated with different batches were pooled, and glycerol was added to this to a final concentration of 10%. Then, this was divided into each 1 ml, and stored at -80˚C.

Example 8 (Selection of compound which modulates c-Jun $NH_2$-terminal kinase activity)

Selection of a compound which modulates the activity of a c-Jun $NH_2$-terminal kinase was performed in a system where the activity of a c-Jun $NH_2$-terminal kinase which is modulated by adding a test compound to an in vitro reaction system using the aphid c-Jun $NH_2$-terminal kinase prepared in Example 7 is measured and evaluated.

Measurement of the activity of the aphid c-Jun $NH_2$-terminal kinase was performed using IMAP screening Express Kit with progressive Binding system (manufactured by Molecular Device) according to a method described in the manual annexed to the system and employing, as a substrate, a peptide comprising the amino acid sequence of

SEQ ID NO: 13 to which biotin was added at the amino-terminus, and a peptide comprising the amino acid sequence of SEQ ID NO: 14 in which the carboxyl-terminus was fluorescently labeled.

For measuring the activity, the activity of the aphid c-Jun $NH_2$-terminal kinase was measured when a test compound dissolved in DMSO was contained to a final concentration of 10 μM. In addition, the activity of the aphid c-Jun $NH_2$-terminal kinase was measured when DMSO was contained in place of a test compound. Then, a ratio (%) of a measured value of the activity of the aphid c-Jun $NH_2$-terminal kinase when a test compound dissolved in DMSO was contained, relative to a measured value of the activity of aphid c-Jun $NH_2$-terminal kinase when DMSO was contained in place of the test compound was calculated, and a value obtained by subtracting the calculated value from 100% was adopted as an inhibition degree (%). Results in each test compound are shown in Table 4 in Example 9 together with results of Example 9.

The activity of aphid c-Jun $NH_2$-terminal kinase was measured when a test compound dissolved in DMSO was contained to a final concentration of each concentration of 100 μM, 30 μM, 10 μM, 3 μM, 1 μM, 0.3 μM, 0.1 μM or 0.03 μM. $IC_{50}$ (μM) was calculated from the result of each concentration at each test compound using a concentration-dependent test analyzing software XL fit (manufactured by idbs). Results are shown in Table 5 in Example 10 together with results of Example 9.

Example 9 (Pesticidal activity test)

A sterilized artificial feed having the following composition (Table 3) was prepared. Then, according to the same manner as that of the method described in Handbook of Insect Rearing Vol. 1 (Elsevier Science Publisers 1985) pp. 35 to pp. 36 except that a test compound dissolved in DMSO to a final concentration of 640 μM was added at 0.5 % volume of the artificial feed, and components were mixed, Aphis gossypii was reared. Six days after rearing, the number of surviving Aphis gossypii was investigated, and an entity exhibiting a significant controlling value (e.g. controlling value of 30% or more) was determined to have pesticidal activity by obtaining a controlling value by the following equation.

$$\text{Controlling value (\%)} = \{1-(Cb \times Tai)/(Cai \times Tb)\} \times 100$$

Letters in the equation represent the following meanings. Cb: Number of surviving worms before treatment in non-treated section

Cai: Number of surviving worms at observation in non-treated section

Tb: Number of surviving worms before treatment in treated section Tai: Number of surviving worms at observation in treated section

Results are shown in Table 4 in Example 9 together with results of Example 8.

Table 3

| Amino acid | (mg/100 ml) | Vitamins | (mg/100 ml) |
|---|---|---|---|
| L-alanine | 100.0 | Ascorbic acid | 100.0 |
| L-arginine | 275.0 | Biotin | 0.1 |
| L-asparagine | 550.0 | Calcium pantothenate | 5.0 |
| L-aspartic acid | 140.0 | Choline chloride | 50.0 |
| L-cysteine (hydrochloride) | 40.0 | Inositol | 50.0 |
| L-glutamic acid | 140.0 | Nicotinic acid | 10.0 |
| L-glutamine | 150.0 | Thiamine | 2.5 |
| L-glycine | 80.0 | | |
| L-histidine | 80.0 | Others | (mg/100 ml) |
| L-isoleucine | 80.0 | Sucrose | 12500.0 |
| L-leucine | 80.0 | Dipotassium hydrogen phosphate | 1500.0 |
| L-lysine (hydrochloride) | 120.0 | Magnesium sulfate | 123.0 |

(continued)

| Amino acid | (mg/100 ml) | Vitamins | (mg/100 ml) |
|---|---|---|---|
| L-methionine | 80.0 | Cupric chloride | 0.2 |
| L-phenylalanine | 40.0 | Ferric chloride | 11.0 |
| L-proline | 80.0 | Manganese chloride | 0.4 |
| L-serine | 80.0 | Zinc sulfate (anhydrous) | 0.8 |
| L-threonine | 140.0 | | |
| L-tryptophan | 80.0 | Adjusted to pH 6.8 | |
| L-tyrosine | 40.0 | | |
| L-valine | 80.0 | | |

Table 4

| Compound name | Structure | Activity of inhibiting c-Jun $NH_2$-terminal kinase activity (inhibition degree (%) at 10 $\mu$M addition) | Determination result of pesticidal activity |
|---|---|---|---|
| Anthra[1,9-CD]p yrazole-6 (2H)-one | 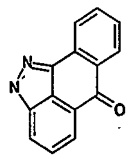 | 83 | Presence of pesticidal activity |
| Arcyriarubin A | 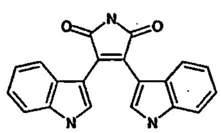 | 73 | Presence of pesticidal activity |
| 4-amino-1-tert-butyl-3-(1-naph thyl)pyrazolo (3,4-d) pyrimidine | 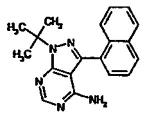 | 68 | Presence of pesticidal activity |

Example 10 (Pesticidal activity test)

According to the same manner as that of Example 9 except that a test compound dissolved in DMSO to a final concentration of 50 ppm was added, pesticidal activity test was performed, and results are shown in Table 5 in Example 10 together with results of Example 8.

Table 5

| Compound name | Structure | Activity of inhibiting c-Jun $NH_2$-terminal kinase activity (IC50, (μM)) | Determination result of pesticidal activity |
|---|---|---|---|
| PH09940 | 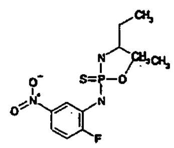 | 35.21 | Presence of pesticidal activity |
| DE01948 | 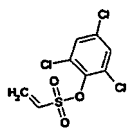 | 21.18 | Presence of pesticidal activity |
| DF06524 | 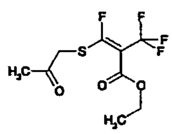 | 47.86 | Presence of pesticidal activity |
| DB04029 | 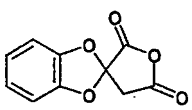 | > 100 | Absence of pesticidal activity |
| DC05429 | 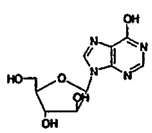 | > 100 | Absence of pesticidal activity |
| DD08068 | 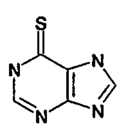 | > 100 | Absence of pesticidal activity |
| DD08100 | 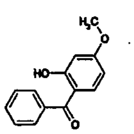 | > 100 | Absence of pesticidal activity |

Industrial Applicability

According to the present invention, it becomes possible to provide a more target-based approach of screening agricultural chemicals, whereby compounds are screened against a specific target that has been identified as biologically and/or physiologically relevant with intent of chemically interfering with the target site to control insects or other pest organisms.

Free Text in Sequence Listing

SEQ ID NO: 3
Designed oligonucleotide primer for PCR
SEQ ID NO: 4

Designed oligonucleotide primer for PCR
SEQ ID NO: 5
Designed oligonucleotide primer for PCR
SEQ ID NO: 6
Designed oligonucleotide primer for PCR
SEQ ID NO: 7
Designed oligonucleotide primer for PCR
SEQ ID NO: 8
Designed oligonucleotide primer for PCR
SEQ ID NO: 9
Designed oligonucleotide primer for PCR
SEQ ID NO: 10
Designed oligonucleotide primer for PCR
SEQ ID NO: 11
Designed oligonucleotide primer for PCR
SEQ ID NO: 12 Designed oligonucleotide primer for PCR
SEQ ID NO: 13
Designed peptide for JNK substrate
SEQ ID NO: 14
Designed peptide for JNK substrate

## Claims

1. A method for assaying pesticidal activity of a test substance, which comprises:

(1) a first step of measuring the activity of a c-Jun $NH_2$-terminal kinase selected from the following group A in a reaction system in which said c-Jun $NH_2$-terminal kinase contacts with a test substance and the peptide of SEQ ID NO: 14; and

(2) a second step of evaluating the pesticidal activity of said test substance based on the difference obtained by comparing the activity measured in the first step with the activity of a control, wherein said test substance is evaluated as having pesticidal activity when it has been identified that said test substance has an ability to inhibit the reaction of the c-Jun $NH_2$-terminal kinase with the peptide of SEQ ID NO: 14:

< group A>

(a) a protein comprising the amino acid sequence of SEQ ID NO: 1;
(b) a protein comprising an amino acid sequence with deletion, addition or substitution of one or more amino acids in the amino acid sequence of SEQ ID NO: 1, wherein said protein has c-Jun $NH_2$-terminal kinase activity;
(c) a protein comprising an amino acid sequence that has sequence identity of 83% or more to the amino acid sequence of SEQ ID NO: 1, wherein said protein has c-Jun $NH_2$-terminal kinase activity;
(d) a protein comprising the amino acid sequence encoded by the nucleotide sequence of SEQ ID NO: 2;
(e) a protein comprising an amino acid sequence encoded by a nucleotide sequence that has sequence identity of 65% or more to the nucleotide sequence of SEQ ID NO: 2, wherein said protein has c-Jun $NH_2$-terminal kinase activity;
(f) a protein comprising an amino acid sequence encoded by a polynucleotide, wherein said polynucleotide hybridizes under a stringent condition to a polynucleotide comprising a nucleotide sequence complementary to the nucleotide sequence of SEQ ID NO: 2, and wherein said protein has c-Jun $NH_2$-terminal kinase activity;
(g) a protein comprising an amino acid sequence of an insect c-Jun $NH_2$-terminal kinase; and
(h) a protein comprising an amino acid sequence of a cotton aphid c-Jun $NH_2$-terminal kinase.

**2.** A method for screening a pesticidal substance, which comprises selecting a substance having the pesticidal activity that is evaluated by the method according to claim 1, wherein the substance is selected (a) when the substance has an ability to inhibit the reaction of the c-Jun $NH_2$-terminal kinase with the peptide of SEQ ID NO: 14 in a cell-free system, wherein in the presence of said substance of 10 micro M or more the activity of the c-Jun $NH_2$-terminal kinase is lower than that in the absence of said substance; or (b) when the substance has an ability to inhibit the reaction of the c-Jun $NH_2$-terminal kinase with the peptide of SEQ ID NO: 14 in a cell-free system with an $IC_{50}$ of 100 micro M or less.

**3.** An insect c-Jun $NH_2$-terminal kinase comprising an amino acid sequence selected from the following group B:

<group B>

(a) the amino acid sequence of SEQ ID NO: 1;
(b) an amino acid sequence with deletion, addition or substitution of one or more amino acids in the amino acid sequence of SEQ ID NO: 1, wherein said amino acid sequence has c-Jun $NH_2$-terminal kinase activity;
(c) an amino acid sequence that has sequence identity of 95% or more to the amino acid sequence of SEQ ID NO: 1, wherein said amino acid sequence has c-Jun $NH_2$-terminal kinase activity;
(d) the amino acid sequence encoded by the nucleotide sequence of SEQ ID NO: 2;
(e) an amino acid sequence encoded by a nucleotide sequence that has sequence identity of 75 % or more to the nucleotide sequence of SEQ ID NO: 2, wherein said amino acid sequence has c-Jun $NH_2$-terminal kinase activity; and
(f) an amino acid sequence encoded by a polynucleotide, wherein said polynucleotide hybridizes under a stringent condition to a polynucleotide comprising a nucleotide sequence complementary to the nucleotide sequence of SEQ ID NO: 2, wherein said amino acid sequence has c-Jun $NH_2$-terminal kinase activity.

**4.** Use of an insect c-Jun $NH_2$-terminal kinase as a reagent that provides an indicator to evaluate pesticidal activity.

**5.** Use according to claim 4, wherein the c-Jun $NH_2$-terminal kinase is that of claim 3.

**6.** A polynucleotide which comprises a nucleotide sequence encoding an amino acid sequence of a c-Jun $NH_2$-terminal kinase according to claim 3.

**7.** A polynucleotide according to claim 6, which comprises the nucleotide sequence of SEQ ID NO: 2.

**8.** A polynucleotide which comprises a nucleotide sequence complementary to a nucleotide sequence of a polynucleotide according to claim 6 or 7.

**9.** A polynucleotide comprising a partial nucleotide sequence of a polynucleotide according to claim 6 or 7 or a nucleotide sequence complementary to said partial nucleotide sequence, which comprises a nucleotide sequence of SEQ ID

NO: 3 or 4.

**10.** A method for obtaining a polynucleotide comprising a nucleotide sequence encoding an amino acid sequence of a c-Jun $NH_2$-terminal kinase, which comprises:

a step of amplifying a desired polynucleotide by polymerase chain reaction using as a primer a polynucleotide according to claim 9;
a step of identifying the desired polynucleotide amplified; and
a step of recovering the identified polynucleotide.

**11.** A method for obtaining a polynucleotide comprising a nucleotide sequence encoding an amino acid sequence of a c-Jun $NH_2$-terminal kinase, which comprises:

a step of detecting a desired polynucleotide by hybridization using as a probe a polynucleotide according to claim 8 or 9;
a step of identifying the desired polynucleotide detected; and
a step of recovering the identified polynucleotide.

**12.** A circular polynucleotide comprising a nucleotide sequence of a polynucleotide according to claim 6 or 7, wherein said nucleotide sequence is operably linked to a baculovirus promoter.

**13.** A circular polynucleotide according to claim 12, wherein said promoter is a polyhedrin gene promoter.

**14.** A circular polynucleotide according to claim 12 or 13, wherein said polynucleotide comprises a replication origin for autonomous replication in a host cell.

**15.** A circular polynucleotide according to claim 12, 13 or 14, wherein said polynucleotide comprises a nucleotide sequence of a baculovirus shuttle vector and capable of propagating as a virus in an insect cell.

**16.** A method for producing a circular polynucleotide, which comprises ligating a polynucleotide according to claim 6 or 7 into a vector.

**17.** A transformant in which a polynucleotide according to claim 6 or 7 is introduced, wherein the transformant is a eucaryotic or a procaryotic cell.

**18.** A transformant according to claim 17, wherein said transformant is a transformed insect cell.

**19.** A method for producing a transformant, which comprises introducing a polynucleotide according to claim 6 or 7 into a host cell.

**20.** A recombinant baculovirus comprising within its genome a polynucleotide according to claim 6 or 7.

**21.** A method for producing a c-Jun $NH_2$-terminal kinase, which comprises a step of culturing the transformant according to claim 17 or 18 and recovering a produced c-Jun $NH_2$-terminal kinase.

**22.** Use of a c-Jun $NH_2$-terminal kinase according to claim 4 or a polynucleotide according to any one of claims 6 to 7 and 9 as an experimental tool for screening a pesticidal substance.

## Patentansprüche

**1.** Verfahren zum Untersuchen auf schädlingsbekämpfende Aktivität einer Testsubstanz, das umfasst:

(1) einen ersten Schritt der Messung der Aktivität einer c-Jun-$NH_2$-terminalen Kinase ausgewählt aus der folgenden Gruppe A in einem Reaktionssystem, in dem die c-Jun-$NH_2$-terminale Kinase in Kontakt mit einer Testsubstanz und dem Peptid von SEQ ID NO:14 kommt; und
(2) einen zweiten Schritt der Evaluierung der schädlingsbekämpfenden Aktivität der Testsubstanz basierend auf dem Unterschied, der durch den Vergleich der im ersten Schritt gemessenen Aktivität mit der Aktivität einer

Kontrolle erhalten wird, wobei die Testsubstanz bewertet wird, schädlingsbekämpfende Aktivität zu haben, wenn erkannt worden ist, dass die Testsubstanz eine Fähigkeit hat, die Reaktion der c-Jun-$NH_2$-terminalen Kinase mit dem Peptid von SEQ ID NO:14 zu inhibieren:

<Gruppe A>

(a) ein Protein umfassend die Aminosäuresequenz von SEQ ID NO:1;
(b) ein Protein umfassend eine Aminosäuresequenz mit Deletion, Addition oder Substitution einer oder mehrerer Aminosäuren in der Aminosäuresequenz von SEQ ID NO:1, wobei das Protein c-Jun-$NH_2$-terminale Kinaseaktivität hat;
(c) ein Protein umfassend eine Aminosäuresequenz, die eine Sequenzidentität von 83% oder mehr zu der Aminosäuresequenz von SEQ ID NO:1 hat, wobei das Protein c-Jun-$NH_2$-terminale Kinaseaktivität hat;
(d) ein Protein umfassend die Aminosäuresequenz, die durch die Nucleotidsequenz von SEQ ID NO: 2 codiert wird;
(e) ein Protein umfassend eine Aminosäuresequenz, die durch eine Nucleotidsequenz codiert wird, die eine Sequenzidentität von 65% oder mehr zu der Nucleotidsequenz von SEQ ID NO:2 hat, wobei das Protein c-Jun-$NH_2$-terminale Kinaseaktivität hat;
(f) ein Protein umfassend eine Aminosäuresequenz, die durch ein Polynucleotid codiert wird, wobei das Polynucleotid unter einer stringenten Bedingung an ein Polynucleotid hybridisiert, das eine Nucleotidsequenz umfasst, die komplementär zu der Nucleotidsequenz von SEQ ID NO:2 ist, und wobei das Protein c-Jun-$NH_2$-terminale Kinaseaktivität hat;
(g) ein Protein umfassend eine Aminosäuresequenz einer Insekten-c-Jun-$NH_2$-terminalen Kinase; und
(h) ein Protein umfassend eine Aminosäuresequenz einer Baumwoll-Blattlaus-c-Jun-$NH_2$-terminalen Kinase.

**2.** Verfahren zum Durchmustern einer schädlingsbekämpfenden Substanz, das das Auswählen einer Substanz umfasst, die die schädlingsbekämpfende Aktivität hat, die durch das Verfahren gemäß Anspruch 1 evaluiert wird, wobei die Substanz ausgewählt wird, (a) wenn die Substanz eine Fähigkeit hat, die Reaktion der c-Jun-$NH_2$-terminalen Kinase mit dem Peptid von SEQ ID NO:14 in einem zellfreien System zu inhibieren, wobei die Aktivität der c-Jun-$NH_2$-terminalen Kinase in Gegenwart von 10 micro M oder mehr der Substanz geringer ist als in der Abwesenheit der Substanz; oder (b) wenn die Substanz eine Fähigkeit hat, die Reaktion der c-Jun-$NH_2$-terminalen Kinase mit dem Peptid von SEQ ID NO:14 in einem zellfreien System mit einem $IC_{50}$ von 100 micro M oder weniger zu inhibieren.

**3.** Insekten-c-Jun-$NH_2$-terminale Kinase umfassend eine Aminosäuresequenz ausgewählt aus der folgenden Gruppe B:

<Gruppe B>

(a) die Aminosäuresequenz von SEQ ID NO:1;
(b) eine Aminosäuresequenz mit Deletion, Addition oder Substitution einer oder mehrerer Aminosäuren in der Aminosäuresequenz von SEQ ID NO:1, wobei die Aminosäuresequenz eine c-Jun-$NH_2$-terminale Kinaseaktivität hat;
(c) eine Aminosäuresequenz, die eine Sequenzidentität von 95% oder mehr zu der Aminosäuresequenz von SEQ ID NO:1 hat, wobei die Aminosäuresequenz c-Jun-$NH_2$-terminale Kinaseaktivität hat;
(d) die Aminosäuresequenz, die durch die Nucleotidsequenz von SEQ ID NO:2 codiert wird;
(e) eine Aminosäuresequenz, die durch eine Nucleotidsequenz codiert wird, die eine Sequenzidentität von 75% oder mehr zu der Nucleotidsequenz von SEQ ID NO:2 hat, wobei die Aminosäuresequenz eine c-Jun-$NH_2$-terminale Kinaseaktivität hat; und
(f) eine Aminosäuresequenz codiert durch ein Polynucleotid, wobei das Polynucleotid unter einer stringenten Bedingung an ein Polynucleotid hybridisiert, das eine Nucleotidsequenz umfasst, die zu der Nucleotidsequenz von SEQ ID NO:2 komplementär ist, wobei die Aminosäuresequenz c-Jun-$NH_2$-terminale Kinaseaktivität hat.

**4.** Verwendung einer Insekten-c-Jun-$NH_2$-terminalen Kinase als ein Reagenz, das einen Indikator bereitstellt, um schädlingsbekämpfende Aktivität zu evaluieren.

**5.** Verwendung gemäß Anspruch 4, wobei die c-Jun-$NH_2$-terminale Kinase die von Anspruch 3 ist.

**6.** Polynucleotid, das eine Nucleotidsequenz umfasst, die eine Aminosäuresequenz einer c-Jun-$NH_2$-terminalen Kinase gemäß Anspruch 3 codiert.

**7.** Polynucleotid gemäß Anspruch 6, das die Nucleotidsequenz von SEQ ID NO:2 umfasst.

**8.** Polynucleotid, das eine Nucleotidsequenz komplementär zu einer Nucleotidsequenz eines Polynucleotids gemäß Anspruch 6 oder 7 umfasst.

**9.** Polynucleotid umfassend eine Teil-Nucleotidsequenz eines Polynucleotids gemäß Anspruch 6 oder 7 oder eine Nucleotidsequenz, die komplementär zu dieser Teil-Nucleotidsequenz ist, die eine Nucleotidsequenz von SEQ ID NO:3 oder 4 umfasst.

**10.** Verfahren zum Erhalten eines Polynucleotids, umfassend eine Nucleotidsequenz, die eine Aminosäuresequenz einer c-Jun-$NH_2$-terminalen Kinase codiert, das umfasst:

einen Schritt der Amplifikation eines gewünschten Polynucleotids durch Polymerasekettenreaktion unter Verwendung eines Polynucleotids gemäß Anspruch 9 als Primer;
einen Schritt der Identifizierung des amplifizierten gewünschten Polynucleotids; und
einen Schritt der Gewinnung des identifizierten Polynucleotids.

**11.** Verfahren zum Erhalten eines Polynucleotids, umfassend eine Nucleotidsequenz, die eine Aminosäuresequenz einer c-Jun-$NH_2$-terminalen Kinase codiert, das umfasst:

einen Schritt des Nachweises eines gewünschten Polynucleotids durch Hybridisierung unter Verwendung eines Polynucleotids gemäß Anspruch 8 oder 9 als Sonde;
einen Schritt der Identifizierung des gewünschten nachgewiesenen Polynucleotids; und
einen Schritt der Gewinnung des identifizierten Polynucleotids.

**12.** Zirkuläres Polynucleotid, umfassend eine Nucleotidsequenz eines Polynucleotids gemäß Anspruch 6 oder 7, wobei die Nucleotidsequenz funktionell mit einem Baculoviruspromotor verbunden ist.

**13.** Zirkuläres Polynucleotid gemäß Anspruch 12, wobei der Promotor ein Polyhedrin-Gen-Promotor ist.

**14.** Zirkuläres Polynucleotid gemäß Anspruch 12 oder 13, wobei das Polynucleotid einen Replikationsursprung für die autonome Replikation in einer Wirtszelle umfasst.

**15.** Zirkuläres Polynucleotid gemäß Anspruch 12, 13 oder 14, wobei das Polynucleotid eine Nucleotidsequenz eines Baculovirus-Shuttlevektors umfasst und fähig ist, sich als Virus in einer Insektenzelle zu vermehren.

**16.** Verfahren zur Erzeugung eines zirkulären Polynucleotids, das das Ligieren eines Polynucleotids gemäß Anspruch 6 oder 7 in einen Vektor umfasst.

**17.** Transformante, in die ein Polynucleotid gemäß Anspruch 6 oder 7 eingeführt ist, wobei die Transformante eine eukaryotische oder eine prokaryotische Zelle ist.

**18.** Transformante gemäß Anspruch 17, wobei die Transformante eine transformierte Insektenzelle ist.

**19.** Verfahren zum Erzeugen einer Transformante, das die Einführung eines Polynucleotids gemäß Anspruch 6 oder 7 in eine Wirtszelle umfasst.

**20.** Rekombinantes Baculovirus, umfassend ein Polynucleotid gemäß Anspruch 6 oder 7 innerhalb seines Genoms.

**21.** Verfahren zur Erzeugung einer c-Jun-$NH_2$-terminalen Kinase, das einen Schritt der Züchtung der Transformante gemäß Anspruch 17 oder 18 und Gewinnung einer erzeugten c-Jun-$NH_2$-terminalen Kinase umfasst.

**22.** Verwendung einer c-Jun-$NH_2$-terminalen Kinase gemäß Anspruch 4 oder eines Polynucleotids gemäß einem der Ansprüche 6 bis 7 und 9 als ein experimentelles Werkzeug zur Durchmusterung einer schädlingsbekämpfenden Substanz.

**Revendications**

1. Procédé destiné à doser l'activité antiparasitaire d'une substance test, comprenant :

    (1) une première étape consistant à mesurer l'activité d'une kinase c-Jun $NH_2$-terminale choisie dans le groupe A suivant dans un système réactionnel dans lequel ladite kinase c-Jun $NH_2$-terminale entre en contact avec une substance test et le peptide de la SEQ ID No. : 14 ; et
    (2) une seconde étape consistant à évaluer l'activité antiparasitaire de ladite substance test en se basant sur la différence obtenue en comparant l'activité mesurée lors de la première étape avec l'activité d'un composé témoin, ladite substance test étant évaluée comme ayant une activité antiparasitaire lorsqu'il a été identifié que ladite substance test présente une capacité à inhiber la réaction de la kinase c-Jun $NH_2$-terminale avec le peptide de la SEQ ID No. : 14 ;
    < groupe A >

        (a) une protéine comprenant la séquence d'acides aminés de la SEQ ID No. : 1 ;
        (b) une protéine comprenant une séquence d'acides aminés présentant une délétion, addition ou substitution d'un ou de plusieurs acides aminés dans la séquence d'acides aminés de la SEQ ID No. : 1, ladite protéine présentant une activité de kinase c-Jun $NH_2$-terminale ;
        (c) une protéine comprenant une séquence d'acides aminés présentant une identité de séquence de 83 % ou plus avec la séquence d'acides aminés de la SEQ ID No. : 1, ladite protéine présentant une activité de kinase c-Jun $NH_2$-terminale ;
        (d) une protéine comprenant la séquence d'acides aminés codée par la séquence nucléotidique de la SEQ ID No. : 2 ;
        (e) une protéine comprenant une séquence d'acides aminés codée par une séquence nucléotidique présentant une identité de séquence de 65 % ou plus avec la séquence nucléotidique de la SEQ ID No. : 2, ladite protéine présentant une activité de kinase c-Jun $NH_2$-terminale ;
        (f) une protéine comprenant une séquence d'acides aminés codée par un polynucléotide, ledit polynucléotide s'hybridant dans une condition stringente à un polynucléotide comprenant une séquence nucléotidique complémentaire de la séquence nucléotidique de SEQ ID No. : 2, et ladite protéine présentant une activité de kinase c-Jun $NH_2$-terminale ;
        (g) une protéine comprenant une séquence d'acides aminés d'une kinase c-Jun $NH_2$-terminale d'insecte ; et
        (h) une protéine comprenant une séquence d'acides aminés d'une kinase c-Jun $NH_2$-terminale de puceron du cotonnier.

2. Procédé de criblage à la recherche d'une substance antiparasitaire, comprenant l'étape consistant à choisir une substance ayant l'activité antiparasitaire qui est évaluée par le procédé selon la revendication 1, la substance étant choisie (a) lorsque la substance présente une capacité à inhiber la réaction de la kinase c-Jun $NH_2$-terminale avec le peptide de la SEQ ID No. : 14 dans un système acellulaire, dans lequel, en présence de ladite substance à une concentration supérieure ou égale à 10 microM, l'activité de la kinase c-Jun $NH_2$-terminale est plus faible qu'en l'absence de ladite substance ; ou (b) lorsque la substance présente une capacité à inhiber la réaction de la kinase c-Jun $NH_2$-terminale avec le peptide de la SEQ ID No. : 14 dans un système acellulaire avec une $CI_{50}$ inférieure ou égale à 100 microM.

3. Kinase c-Jun $NH_2$-terminale d'insecte comprenant une séquence d'acides aminés choisie dans le groupe B suivant :

    < groupe B >

        (a) la séquence d'acides aminés de la SEQ ID No. : 1 ;
        (b) une séquence d'acides aminés présentant une délétion, addition ou substitution d'un ou de plusieurs acides aminés dans la séquence d'acides aminés de la SEQ ID No. : 1, ladite séquence d'acides aminés présentant une activité de kinase c-Jun $NH_2$-terminale ;
        (c) une séquence d'acides aminés présentant une identité de séquence de 95 % ou plus avec la séquence d'acides aminés de la SEQ ID No. : 1, ladite séquence d'acides aminés présentant une activité de kinase c-Jun $NH_2$-terminale ;
        (d) la séquence d'acides aminés codée par la séquence nucléotidique de la SEQ ID No. : 2 ;
        (e) une séquence d'acides aminés codée par une séquence nucléotidique présentant une identité de séquence de 75 % ou plus avec la séquence nucléotidique de la SEQ ID No. : 2, ladite séquence d'acides aminés présentant une activité de kinase c-Jun $NH_2$-terminale ; et

(f) une séquence d'acides aminés codée par un polynucléotide, ledit polynucléotide s'hybridant dans une condition stringente à un polynucléotide comprenant une séquence nucléotidique complémentaire de la séquence nucléotidique de SEQ ID No. : 2, ladite séquence d'acides aminés présentant une activité de kinase c-Jun $NH_2$-terminale.

**4.** Utilisation d'une kinase c-Jun $NH_2$-terminale d'insecte comme réactif fournissant une indication dans l'évaluation de l'activité antiparasitaire.

**5.** Utilisation selon la revendication 4, dans laquelle la kinase c-Jun $NH_2$-terminale est celle de la revendication 3.

**6.** Polynucléotide comprenant une séquence nucléotidique codant pour une séquence d'acides aminés d'une kinase c-Jun $NH_2$-terminale selon la revendication 3.

**7.** Polynucléotide selon la revendication 6, comprenant la séquence nucléotidique de la SEQ ID No. : 2.

**8.** Polynucléotide comprenant une séquence nucléotidique complémentaire d'une séquence nucléotidique d'un polynucléotide selon la revendication 6 ou 7.

**9.** Polynucléotide comprenant une séquence nucléotidique partielle d'un polynucléotide selon la revendication 6 ou 7 ou une séquence nucléotidique complémentaire de ladite séquence nucléotidique partielle, comprenant une séquence nucléotidique de la SEQ ID No. : 3 ou 4.

**10.** Procédé destiné à obtenir un polynucléotide comprenant une séquence nucléotidique codant pour une séquence d'acides aminés d'une kinase c-Jun $NH_2$-terminale, comprenant :

une étape d'amplification d'un polynucléotide souhaité par une réaction en chaîne par polymérase en utilisant comme amorce un polynucléotide selon la revendication 9 ;
une étape d'identification du polynucléotide amplifié souhaité ; et
une étape de récupération du polynucléotide identifié.

**11.** Procédé destiné à obtenir un polynucléotide comprenant une séquence nucléotidique codant pour une séquence d'acides aminés d'une kinase c-Jun $NH_2$-terminale, comprenant :

une étape de détection d'un polynucléotide souhaité par hybridation en utilisant comme sonde un polynucléotide selon la revendication 8 ou 9 ;
une étape d'identification du polynucléotide détecté souhaité ; et
une étape de récupération du polynucléotide identifié.

**12.** Polynucléotide circulaire comprenant une séquence nucléotidique d'un polynucléotide selon la revendication 6 ou 7, dans lequel ladite séquence nucléotidique est liée de manière fonctionnelle à un promoteur de baculovirus.

**13.** Polynucléotide circulaire selon la revendication 12, dans lequel ledit promoteur est un promoteur du gène de la polyhédrine.

**14.** Polynucléotide circulaire selon la revendication 12 ou 13, ledit polynucléotide comprenant une origine de réplication pour une réplication autonome dans une cellule hôte.

**15.** Polynucléotide circulaire selon la revendication 12, 13 ou 14, ledit polynucléotide comprenant une séquence nucléotidique d'un vecteur navette de baculovirus et étant capable de se propager comme virus dans une cellule d'insecte.

**16.** Procédé de production d'un polynucléotide circulaire, comprenant l'étape consistant à ligaturer un polynucléotide selon la revendication 6 ou 7 à l'intérieur d'un vecteur.

**17.** Transformé dans lequel est introduit un polynucléotide selon la revendication 6 ou 7, le transformé étant une cellule eucaryote ou procaryote.

**18.** Transformé selon la revendication 17, ledit transformé étant une cellule transformée d'insecte.

**19.** Procédé de production d'un transformé, comprenant l'étape consistant à introduire un polynucléotide selon la revendication 6 ou 7 à l'intérieur d'une cellule hôte.

**20.** Baculovirus recombiné comprenant dans son génome un polynucléotide selon la revendication 6 ou 7.

**21.** Procédé de production d'une kinase c-Jun $NH_2$-terminale, comprenant une étape consistant à cultiver le transformé selon la revendication 17 ou 18 et à récupérer une kinase c-Jun $NH_2$-terminale produite.

**22.** Utilisation d'une kinase c-Jun $NH_2$-terminale selon la revendication 4 ou d'un polynucléotide selon l'une quelconque des revendications 6 à 7 et 9 comme outil expérimental destiné à cribler une substance antiparasitaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0009682 A
- JP 2005183032 A


### Non-patent literature cited in the description


- **Sluss et al.** *Genes Dev.,* 1996, vol. 10 (21), 2745-58
- **McEven et al.** *Development,* 2000, vol. 127 (16), 3607-17
- **Adachi-Yamadaet.** *Nature,* 1999, vol. 400 (6740), 166-9
- **Noselli et al.** *Curr Opin Genet Dev.,* 1999, vol. 9 (4), 466-72
- **Fowler Ann et al.** *Analytical Biochemistry,* 2002, vol. 308, 223-231
- **Bennett BL et al.** *Proc Natl Acad Sci USA.,* 20 November 2001, vol. 98 (24), 13681-6
- Handbook of Insect Rearing. Elsevier Science Publisers, 1985, vol. 1, 35-36
- *Nucleic Acids Res.,* 1984, vol. 12, 9441-9456
- *Method in Molecular Biology,* 1994, 97-112
- **Pearson ; Lipman.** *Proc. Natl. Acad. Sci. USA,* 1988, vol. 4, 2444-2448
- **Altschul et al.** *Molecular Biology,* 1990, vol. 215, 403-410
- **Thompson ; Higgins ; Gibson.** *Nucleic Acid Research,* 1994, vol. 22, 4673-4680
- **Lipman, D. J. ; Pearson, W.R.** *Science,* 1985, vol. 227, 1435-1441
- **Sambrook J. ; Frisch E. F. ; Maniatis T.** Molecular Cloning. Cold Spring Harbor Laboratory press
- Molecular Biology. John Wiley & Sons, 1989, 6.3.1-6.3.6
- **Altschul et al.** *Journal of Molecular Biology,* 1990, vol. 215, 403-410
- **Higgins ; Gibson.** *Nucleic Acid Research,* 1994, vol. 22, 4673-4680
- Molecular Cloning: A Laboratory Manual. Cold Spring Harbor Laboratory Press, 1989
- Current Protocols In Molecular Biology. John Wiley & Sons,Inc, 1987
- **Maxam,A.M ; W.Gilbert.** *Proc.Natl.Acad.Sci.USA,* 1977, vol. 74, 560
- **Sanger,F. ; A.R.Coulson.** *J.Mol.Biol.,* 1975, vol. 94, 441
- **Sanger,F ; Nicklen ; A.R.Coulson.** *Proc.Natl.Acad.Sci.USA,* 1977, vol. 74, 5463
- **Harris ; Polayes.** *Focus,* 1997, vol. 19, 6-8
- **Pijlman et al.** *Journal of General Virology,* 2003, vol. 84, 2669-2678
- **Kitts.** *Cytotechnology,* 1996, vol. 20, 111-123
- **Ian N. Foltz et al.** *The Journal of Biological Chemistry,* 1998, vol. 273, 9344-9351